(19) Europäisches Patentamt
European Patent Office
Office européen des brevets

(11) **EP 4 484 406 A1**

(12) **EUROPEAN PATENT APPLICATION**
published in accordance with Art. 153(4) EPC

(43) Date of publication:
**01.01.2025 Bulletin 2025/01**

(21) Application number: **23760034.1**

(22) Date of filing: **22.02.2023**

(51) International Patent Classification (IPC):
*C07C 69/40* (2006.01)      *A61K 8/37* (2006.01)
*A61Q 1/00* (2006.01)       *A61Q 17/04* (2006.01)
*A61Q 19/00* (2006.01)      *C07C 67/08* (2006.01)
*C07C 69/30* (2006.01)      *C11C 3/02* (2006.01)

(52) Cooperative Patent Classification (CPC):
**A61K 8/37; A61Q 1/00; A61Q 17/04; A61Q 19/00;
C07C 67/08; C07C 69/30; C07C 69/40; C11C 3/02**

(86) International application number:
**PCT/JP2023/006436**

(87) International publication number:
**WO 2023/163035 (31.08.2023 Gazette 2023/35)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **24.02.2022 JP 2022027108**

(71) Applicant: **THE NISSHIN OILLIO GROUP, LTD.
Tokyo 104-8285 (JP)**

(72) Inventors:
• **SHIBATA Masashi
Hachioji-shi, Tokyo 192-0982 (JP)**

• **TAKETANI Shunsuke
Yokohama-shi, Kanagawa 235-8558 (JP)**
• **SHIMIZU Daisuke
Yokohama-shi, Kanagawa 235-8558 (JP)**
• **KACHI Hisanori
Yokohama-shi, Kanagawa 235-8558 (JP)**
• **IMAI Takahiko
Yokohama-shi, Kanagawa 235-8558 (JP)**

(74) Representative: **Hasegawa, Kan
Patentanwaltskanzlei Hasegawa
Untere Hauptstraße 56
85354 Freising (DE)**

(54) **OLIGOESTER AND COSMETIC CONTAINING SAID OLIGOESTER**

(57)    Provided is an oligoester which is obtained by subjecting the following to an esterification reaction: component (A), a glycerin; component (B), a dicarboxylic acid having 2-12 carbon atoms; and component (C), one or more types of fatty acid selected from the group consisting of caprylic acid, ethylhexanoic acid, isononanoic acid, isopalmitic acid and isostearic acid. The esterification reaction involves esterifying 0.65-0.8 moles of the component (B) and 1.2-1.7 moles of the component (C) relative to 1 mole of the component (A). The oligoester has an acid value of 5 mgKOH/g or less, a saponification value of 420-510 mgKOH/g and a hydroxyl value of 60 mgKOH/g or less.

EP 4 484 406 A1

**Description**

[Technical Field]

The present invention relates to an oligoester, and a cosmetic containing the oligoester.

Priority is claimed on Japanese Patent Application No. 2022-027108, filed February 24, 2022, the content of which is incorporated herein by reference.

[Background Art]

Cosmetics such as lip cosmetics are required to have not only favorable makeup effects such as imparting a glossy finish upon application to the skin, but also excellent usability including favorable spreadability on the skin, good maintenance of the hardness achieved during preparation, and a combination of favorably usability and shape retention over time.

The components of conventional lip cosmetics typified by lipsticks can be broadly classified into pigments or dyes that impart vivid coloration to the lips, waxes that are solid at room temperature and serves as excipient components, and oils that uniformly disperse or dissolve these components, while also endowing functional characteristics such as usage sensation and gloss. In the field of lip cosmetics, adjustment of the mixing ratio between the waxes and liquid oil components is investigated to ensure a combination of the shape retention required for the product and a favorable sensation upon use.

Examples of methods for improving the shape retention and sensation upon use include a technique in which a Fischer-Tropsch wax and another specific wax are combined to improve the usability and the high-temperature stability (Patent Document 1), and a technique in which an oil having a melting point of 75°C or higher is combined with a dimer acid, an N-long chain acyl-modified amino acid diester, and an organic spherical powder (Patent Document 2). However, with these techniques, products capable of fully satisfying the required combination of shape retention and sensation upon use could not be obtained. In addition, the N-long chain acyl-modified amino acid diester must be added in a comparatively large amount to achieve a satisfactory effect, but is relatively expensive. The phytosterols that are added also suffer from high cost, and have limitations in terms of market supply. Moreover, based on the fact that tint-type lipsticks, which undergo a change in hue upon reaction with the moisture in the lips, have garnered a market share, lip cosmetics that undergo a change in texture following application are also in demand.

One important factor required of lip cosmetics is the gloss of the coating film. In order to enhance this gloss, methods for increasing the light reflectance by adding oils having a high refractive index or adding pearl agents are typically used. Further, a bleed technique in which a silicone oil with poor miscibility with the lipstick oil is added to the lip cosmetic, causing the silicone oil to bleed through to the surface of the coating film and create a layer of high gloss (Patent Document 3) is also being used.

For the liquid oils that are added to cosmetics, an ability of maintaining a liquid state at low temperatures with good suppression of crystallization is deemed an important factor in terms of product stability. This is because if the liquid oil within a cosmetic crystallizes at low temperature, the lipstick becomes brittle. Furthermore, it is known that if the liquid oil is added to the cosmetic in an emulsified state, the demulsification can increase the likelihood of problems such as component separation or texture changes.

In those cases where the wax is not completely melted during the heating conducted within the preparation of a lip cosmetic, problems tend to occur such as fluctuations in the product quality caused by component separation upon cooling or unstable wax growth. Further, in those cases where the pigment is not dispersed uniformly, the coloration following application tends to worsen, and therefore pigment dispersibility is also an important function required of the base oil of a lip cosmetic.

On the other hand, oligoesters have been conventionally used in cosmetics for reasons including a better skin moisturizing effect, superior stability, and excellent pigment dispersibility. Example of typical oligoesters added to cosmetics include oligoesters that dissolve in water and have a superior moisturizing effect and a favorable sensation upon use (Patent Document 4), oligoesters that melt at temperatures close to body temperature, suppress moisture diffusion from the skin surface and have a sensation upon use with a reduced oily feeling (Patent Document 5), and oligoesters that serve as a gelling agent or solidifying agent for liquid oils (Patent Document 6). Furthermore, powders that have been surface-treated with an oligoester to provide an excellent sensation upon use and superior pigment dispersibility (Patent Document 7) and two-layer cosmetics containing glyceryl (caprylate/caprate/succinate) (Patent Document 8) are also being developed.

In addition, hydrating oils are also used as the liquid oils added to cosmetics. Hydrating oils exhibit excellent moisturizing properties, and it is expected that addition of these hydrating oils will be able to improve the moisturizing properties of cosmetics (Patent Document 9). However, problems have arisen including cloudiness caused by moisture retention and a deterioration on the external gloss.

[Citation List]

[Patent Documents]

[Patent Document 1]
Japanese Unexamined Patent Application, First Publication No. 2009-234991
[Patent Document 2]
Japanese Unexamined Patent Application, First Publication No. 2015-124160
[Patent Document 3]
Japanese Unexamined Patent Application, First Publication No. 2011-140479
[Patent Document 4]
Japanese Unexamined Patent Application, First Publication No. 2007-137847
[Patent Document 5]
Japanese Unexamined Patent Application, First Publication No. 2006-315975
[Patent Document 6]
Japanese Unexamined Patent Application, First Publication No. Hei 07-126604
[Patent Document 7]
Japanese Unexamined Patent Application, First Publication No. 2018-168141
[Patent Document 8]
Japanese Unexamined Patent Application, First Publication No. 2016-222637
[Patent Document 9]
Japanese Unexamined Patent Application, First Publication No. 2015-48323

[Summary of Invention]

[Technical Problem]

Until now, no oligoester has been developed that not only exhibits an excellent effect of maintaining hardness of the oily solid, but also has excellent pigment dispersibility, and an effect that yields an improvement in the gloss of the applied surface upon contact with moisture.

Accordingly, the present invention has an object of providing an oligoester having an excellent effect of maintaining hardness of the oily solid, superior wax solubility, low-temperature stability and pigment dispersibility, and an effect that yields an improvement in the gloss of the applied surface upon contact with moisture, as well as providing a method for producing the oligoester.

Furthermore, the present invention also has an object of providing a cosmetic containing the oligoester, and having favorable storage stability and excellent adhesion and spreadability upon application to the skin.

[Solution to Problem]

As a result of intensive investigation aimed at achieving the objects described above, the inventors of the present invention discovered that an oligoester obtained by subjecting glycerol, a dicarboxylic acid having 2 to 12 carbon atoms, and a saturated fatty acid having 6 to 24 carbon atoms to an esterification reaction exhibited excellent wax solubility, low-temperature stability and pigment dispersibility, had a superior effect of maintaining hardness of the oily solid, and also had an effect that yielded an improvement in the gloss of the applied surface upon contact with moisture, thus enabling them to complete the present invention.

In other words, the present invention relates to the following aspects.

[1] An oligoester obtained by subjecting a component (A), a component (B) and a component (C) to an esterification reaction, wherein

the esterification reaction involves esterifying 0.65 to 0.8 mol of the component (B) and 1.2 to 1.7 mol of the component (C) relative to one mol of the component (A), and
the oligoester has an acid value of 5 mgKOH/g or less, a saponification value of 420 to 510 mgKOH/g, and a hydroxyl value of 60 mgKOH/g or less.
Component (A): glycerol.
Component (B): a dicarboxylic acid having 2 to 12 carbon atoms.

Component (C): one or more fatty acids selected from the group consisting of caprylic acid, ethylhexanoic acid, isononanoic acid, isopalmitic acid and isostearic acid.

[2] An oligoester obtained by subjecting a component (A), a component (B), a component (C) and a component (D) to an esterification reaction, wherein

the esterification reaction involves esterifying 0.65 to 0.8 mol of the component (B) and 1.2 to 1.7 mol of the combined total of the component (C) and the component (D) relative to one mol of the component (A),
the mass ratio between the component (C) and the component (D), component (C) : component (D), used in the esterification reaction is within a range of 99.9:0.1 to 10:90, and
the oligoester has an acid value of 5 mgKOH/g or less, a saponification value of 420 to 510 mgKOH/g, and a hydroxyl value of 60 mgKOH/g or less.
Component (A): glycerol.
Component (B): a dicarboxylic acid having 2 to 12 carbon atoms.
Component (C): one or more fatty acids selected from the group consisting of caprylic acid, ethylhexanoic acid, isononanoic acid, isopalmitic acid and isostearic acid.
Component (D): one or more fatty acids selected from the group consisting of linear saturated fatty acids having 10 to 24 carbon atoms.

[3] The oligoester according to [2], wherein the component (D) is capric acid.
[4] The oligoester according to any one of [1] to [3], wherein the component (C) is caprylic acid.
[5] The oligoester according to any one of [1] to [4], wherein the component (B) is succinic acid.
[6] An oily composition containing the oligoester of any one of [1] to [5].
[7] The oily composition according to [6], further containing a wax that is solid at 25°C.
[8] The oily composition according to [6] or [7], wherein the composition is a solid.
[9] The oily composition according to [6] or [7], wherein the composition is a semi-solid.
[10] A cosmetic containing the oligoester of any one of [1] to [5].
[11] The cosmetic according to [10], further containing a wax that is solid at 25°C.
[12] The cosmetic according to [10] or [11], wherein the cosmetic is a lip cosmetic.
[13] A stationery product containing the oligoester of any one of [1] to [5].
[14] The stationery product according to [13], wherein the stationery product is a crayon.
[15] A method for producing an oligoester, including conducting an esterification reaction of 0.65 to 0.8 mol of succinic acid and 1.2 to 1.7 mol of a fatty acid relative to one mol of glycerol, wherein

the fatty acid contains caprylic acid and capric acid, and the mass ratio between the caprylic acid and the capric acid, caprylic acid : capric acid, is within a range of 99:1 to 10:90, and
the oligoester has an acid value of 10 mgKOH/g or less, a saponification value of 420 to 510 mgKOH/g, and a hydroxyl value of 60 mgKOH/g or less.

[16] An oily solid hardness maintenance agent containing the oligoester of any one of [1] to [5], and a wax that is solid at 25°C.
[17] A method for maintaining the hardness of an oily solid, including incorporating the oligoester of any one of [1] to [5] into an oily solid containing a wax that is solid at 25°C.
[18] A method for improving the gloss of the applied surface of a cosmetic, the method including incorporating the oligoester of any one of [1] to [5] into the cosmetic.
[19] The method for improving gloss according to [18], wherein the cosmetic is a lip cosmetic.

[Advantageous Effects of Invention]

The oligoester according to the present invention can maintain the hardness of an oily solid over time, can be used to stably produce an oily solid as a result of having superior wax solubility, exhibits excellent low-temperature stability and pigment dispersibility, and has an effect that yields an improvement in the gloss of the applied surface upon contact with moisture. Furthermore, the oligoester can be produced by combining inexpensive raw materials, and therefore the present invention has considerable industrial value.

A cosmetic containing the oligoester exhibits excellent cosmetic performance and stability.

[Description of Embodiments]

In the present invention and the present description, the term "oily solid" means a solid that contains an oily component and has no fluidity at normal temperatures (15 to 25°C) and normal pressure. Furthermore, the term "semi-solid" refers to a property that exhibits viscoelasticity and shape retention, but becomes fluid and can be freely deformed upon application of an external force. The term "semi-solid" can be classified into jelly-like, gel-like, paste-like, ointment-like or the like, depending on the viscosity and water content.

<Oligoester>

The oligoester according to the present invention is obtained by conducting an esterification reaction of 0.65 to 0.8 mol of a component (B) described below and 1.2 to 1.7 mol of a component (C) described below relative to one mol of a component (A) described below, wherein the oligoester has an acid value of 5 mgKOH/g or less, a saponification value of 420 to 510 mgKOH/g, and a hydroxyl value of 60 mgKOH/g or less.

Component (A): glycerol.
Component (B): a dicarboxylic acid having 2 to 12 carbon atoms.
Component (C): one or more fatty acids selected from the group consisting of caprylic acid (n-octanoic acid), ethylhexanoic acid, isononanoic acid, isopalmitic acid and isostearic acid.

The oligoester according to the present invention may also be an esterified product obtained using not only the component (C), but also a component (D) described below, as the fatty acids used in the esterification reaction. Specifically, the oligoester according to the present invention also includes an oligoester obtained by conducting an esterification reaction of 0.65 to 0.8 mol of a component (B) described below and 1.2 to 1.7 mol of a combine total of a component (C) and a component (D) described below relative to one mol of a component (A) described below, wherein the mass ratio between the component (C) and the component (D), component (C) : component (D), used in the esterification reaction is within a range of 99.9:0.1 to 10:90, and the oligoester has an acid value of 5 mgKOH/g or less, a saponification value of 420 to 510 mgKOH/g, and a hydroxyl value of 60 mgKOH/g or less.

Component (A): glycerol.
Component (B): a dicarboxylic acid having 2 to 12 carbon atoms.
Component (C): one or more fatty acids selected from the group consisting of caprylic acid, ethylhexanoic acid, isononanoic acid, isopalmitic acid and isostearic acid.
Component (D): one or more fatty acids selected from the group consisting of linear saturated fatty acids having 10 to 24 carbon atoms.

The oligoester according to the present invention has an action that suppresses any reduction in the hardness of oily solids over time (an oily solid hardness maintenance action). In other words, an oily solid containing the oligoester according to the present invention is able to maintain the initial hardness achieved following production for a longer time period than an oily solid that does not contain the oligoester according to the present invention.
The oligoester according to the present invention exhibits favorable solubility in waxes. Accordingly, the oligoester according to the present invention can be dissolved in a melted wax, and a wax containing the oligoester according to the present invention dispersed uniformly therein can be obtained by cooling the melted wax containing the dissolved oligoester according to the present invention. The oligoester according to the present invention can be dissolved favorably not only in waxes, but also in oily compositions containing a wax. The term "wax" means an oily substance which is solid at 25°C, but melts and becomes a liquid at 120°C.
The oligoester according to the present invention is liquid at 20°C.
Furthermore, the oligoester according to the present invention has favorable low-temperature stability, and crystal precipitation can be suppressed even in low-temperature states. As a result, an oily composition containing the oligoester according to the present invention exhibits good suppression of crystal precipitation even at low temperatures, and can be kept in a stable state.
The oligoester according to the present invention exhibits excellent pigment dispersibility. Accordingly, the dispersibility of a pigment can be improved, and the pigment can be dispersed uniformly throughout an entire oily composition, by incorporating the oligoester according to the present invention in the oily composition containing the pigment.
The oligoester according to the present invention also has an effect that yields an improvement in the gloss of the applied surface upon contact with moisture. This "effect that yields an improvement in the gloss of the applied surface upon contact with moisture" means an effect wherein the gloss of the applied surface visually improves after contact with moisture compared with the state immediately following application.

This effect that yields an improvement in the gloss of the applied surface upon contact with moisture can be confirmed, for example, in the manner described below.

Following application of a thin film of the test oil to the skin, the applied surface is exposed to running water while being rubbed lightly back and forth 20 times, and the applied surface is then removed from the running water. The applied surface is shaken to remove water droplets, and left to stand for 5 minutes to dry. An evaluation is then conducted by comparing the gloss of the applied surface following drying with the gloss of the applied surface immediately following application.

The component (B) used in the present invention is a dicarboxylic acid having 2 to 12 carbon atoms. Examples of the dicarboxylic acid having 2 to 12 carbon atoms include oxalic acid (ethanedioic acid), malonic acid (propanedioic acid), succinic acid (butanedioic acid), glutaric acid (pentanedioic acid), adipic acid (hexanedioic acid), pimelic acid (heptanedioic acid), suberic acid (octanedioic acid), azelaic acid (nonanedioic acid), sebacic acid (decanedioic acid), phthalic acid (benzene-1,2-dicarboxylic acid), isophthalic acid (benzene-1,3-dicarboxylic acid), and terephthalic acid (benzene-1,4-dicarboxylic acid). In terms of ensuring better hardness maintenance, succinic acid is particularly preferred as the component (B) that is one of the raw materials of the oligoester according to the present invention.

The component (C) used in the present invention is one or more fatty acids selected from the group consisting of caprylic acid, ethylhexanoic acid, isononanoic acid, isopalmitic acid and isostearic acid. Caprylic acid is particularly preferred as the component (C) that is one of the raw materials of the oligoester according to the present invention. Furthermore, the oligoester according to the present invention exhibits a better hue and better odor than an oligoester obtained by subjecting an unsaturated fatty acid, the component (A) and the component (B) to an esterification reaction.

The component (D) used in the present invention is one or more fatty acids selected from the group consisting of linear saturated fatty acids having 10 to 24 carbon atoms. Specific examples of these fatty acids include capric acid (n-decanoic acid), lauric acid, myristic acid, palmitic acid, stearic acid, and behenic acid. Capric acid is particularly preferred as the component (D) that is one of the raw materials of the oligoester according to the present invention.

In those cases where both the component (C) and the component (D) are used as fatty acids, the mass ratio between the component (C) and the component (D), component (C) : component (D), used in the esterification reaction is preferably within a range of 99.9:0.1 to 10:90, more preferably from 99:1 to 25:75, and even more preferably from 99:1 to 40:60. The mass ratio of the component (D) relative to the total mass of the component (C) and the component (D) (namely, [mass of component (D)] / ([mass of component (C)]+[mass of component (D)])) may be less than 0.1% by mass, but the mass ratio of the mass of the component (C) relative to the total mass of the component (C) and the component (D) (namely, [mass of component (C)] / ([mass of component (C)]+[mass of component (D)])) is preferably 8 or greater. By ensuring an adequate amount of the component (C), crystal precipitation under low-temperature conditions can be suppressed, and an oligoester having superior low-temperature stability can be obtained.

The acid value of the oligoester according to the present invention is preferably 5 mgKOH/g or less (0 to 5 mgKOH/g). If the acid value is extremely high, there is a possibility that an unpleasant odor may develop. By ensuring that the acid value is 5 mgKOH/g or less, the oligoester according to the present invention lacks any characteristic odor, and can be blended into various products without adversely affecting the product quality.

Adjusting the acid value of the oligoester according to the present invention so that it falls within the above range can be achieved by sampling the reaction product during the esterification reaction, measuring the acid value of the sample, and then halting the esterification reaction once the desired acid value has been reached.

The saponification value of the oligoester according to the present invention is preferably within a range of 420 to 510 mgKOH/g, more preferably from 420 to 490 mgKOH/g, and even more preferably from 430 to 490 mgKOH/g. When the saponification value falls within the above-mentioned range, a favorable effect of maintaining hardness of the oily solid is achieved, the wax solubility, low-temperature stability and pigment dispersibility are favorable, and the gloss of the applied surface upon contact with moisture is also improved.

The saponification value of the oligoester according to the present invention can be adjusted by altering the blend amounts used in the esterification reaction.

The hydroxyl value of the oligoester according to the present invention is preferably 60 mgKOH/g or less, more preferably within a range from 0 to 60 mgKOH/g, even more preferably from 0 to 40 mgKOH/g, still more preferably from 0 to 35 mgKOH/g, and particularly preferably from 0 to 20 mgKOH/g. If the hydroxyl value is extremely high, there is a possibility that the wax solubility may deteriorate. By ensuring that the hydroxyl value is 60 mgKOH/g or less, the oligoester according to the present invention exhibits favorable wax solubility.

The hydroxyl value of the oligoester according to the present invention can be adjusted by altering the blend amounts used in the esterification reaction.

Commercially available products may be used for each of these components (A) to (D).

Glycerol and linear saturated fatty acids are generally available as plant-derived products, whereas succinic acid is mass-produced industrially using a fermentation process. Accordingly, by using succinic acid as the component (B), the oligoester according to the present invention can be produced by combining inexpensive raw materials. Among them, the raw materials of the oligoester according to the present invention are preferably glycerol as the component (A), succinic acid as the component (B), caprylic acid, which is a linear saturated fatty acid, as the component (C), and capric acid as the component (D), from the viewpoints of environmental impact and sustainability of supply.

<Production of the Oligoester by an Esterification Reaction>

The oligoester according to the present invention can be produced by adjusting the amounts of the raw materials used in the esterification reaction to alter the degree of crosslinking and the number of side chains, thus obtaining an oligoester that exhibtis desired effects. Specifically, in the oligoester according to the present invention, the degree of crosslinking in the obtained oligoester can be controlled by altering the blended amounts of the component (A) and the component (B). Furthermore, the degree of crosslinking and the number of added side chains can be controlled by altering the blended amount of the fatty acid of the component (C).

In the esterification reaction used to produce the oligoester according to the present invention, the blended amounts of the component (A), the component (B) and the component (C) are set so that the amount of the component (B) is within a range of 0.65 to 0.8 mol and the amount of the component (C) is within a range of 1.2 to 1.7 mol relative to one mol of the component (A). Furthermore, it is preferable that the amount of the component (B) be within a range of 0.65 to 0.75 mol and the amount of the component (C) be within a range of 1.35 to 1.7 mol relative to one mol of the component (A), and more preferable that the amount of the component (B) be within a range of 0.7 to 0.75 mol and the amount of the component (C) be within a range of 1.35 to 1.6 mol relative to one mol of the component (A).

If the blended amount of the component (B) relative to one mol of the component (A) is less than 0.65 mol, the resulting oligoester does not exhibit an effect of maintaining hardness of the oily solid, and may also not exhibit an effect that yields an improvement in the gloss of the applied surface upon contact with moisture. On the other hand, if the component (B) is used in an amount exceeding 0.8 mol, the crosslinking of the component (A) and the component (B) may proceed excessively, causing cloudiness in the obtained oligoester.

If the blended amount of the component (C) relative to one mol of the component (A) exceeds 1.7 mol, an ester exchange may occur, and the synthesis may sometimes not proceed as far as the desired degree of crosslinking. On the other hand, if the blended amount of the component (C) is less than 1.2 mol, the number of unreacted hydroxyl groups increases, and the wax solubility of the obtained oligoester may worsen.

Similarly, even in those cases where the component (D) is used as a raw material, the degree of crosslinking in the obtained oligoester can be controlled by altering the blended amounts of the component (A) and the component (B). Furthermore, the degree of crosslinking and the number of added side chains can be controlled by altering the blended amounts of the fatty acids of the component (C) and the component (D).

In those cases where the component (D) is used as a raw material, the blended amounts of the component (A), the component (B), the component (C) and the component (D) are set so that the amount of the component (B) is within a range of 0.65 to 0.8 mol, and the combined amount of the component (C) and the component (D) is within a range of 1.2 to 1.7 mol relative to one mol of the component (A). Furthermore, it is preferable that the amount of the component (B) be within a range of 0.65 to 0.75 mol and the combined amount of the component (C) and the component (D) be within a range of 1.35 to 1.7 mol relative to one mol of the component (A), and more preferable that the amount of the component (B) be within a range of 0.7 to 0.75 mol and the combined amount of the component (C) and the component (D) be within a range of 1.35 to 1.6 mol relative to one mol of the component (A).

In those cases where the component (D) is used as a raw material in the esterification reaction, the mass ratio between the blended amounts of the component (C) and the component (D) is preferably within a range from 99.9:0.1 to 10:90, more preferably from 99:1 to 25:75, and even more preferably from 99:1 to 40:60. In the mass ratio between the blended amounts of the component (C) and the component (D), the mass percentage of the component (D) may be less than 0.1. On the other hand, in the mass ratio between the blended amounts of the component (C) and the component (D), if the mass percentage of the component (C) is 8 (namely, the mass ratio of component (C) : component (D) is 8:92) or lower, the obtained oligoester tends to cause crystal precipitation under low-temperature conditions, and the low-temperature stability also worsens, both of which are undesirable.

The oligoester according to the present invention may be produced, for example, by charging 0.65 to 0.8 mol of the component (B) and 1.2 to 1.7 mol of the component (C) relative to one mol of the component (A), and then allowing the reaction to proceed at a temperature within a range of 180 to 240°C, either in the presence or absence of a catalyst. In those cases where the component (D) is also used as a raw material, the oligoester according to the present invention may be produced, for example, by charging 0.65 to 0.8 mol of the component (B) and 1.2 to 1.7 mol of the combination of the component (C) and the component (D) relative to one mol of the component (A), and then allowing the reaction to proceed at a temperature within a range of 180 to 240°C, either in the presence or absence of a catalyst.

Examples of the catalyst which may be used include: acids and alkalis which are typically used in the esterification reactions of alcohols and fatty acids; and other conventionally known catalysts used in the field of organic chemistry. The esterification reaction may be conducted in a solvent that has no adverse effect on the esterification of the alcohol and the fatty acid, or may be conducted in the absence of a solvent. Examples of the solvent include solvents which are typically used in the esterification reactions of alcohols and fatty acid and are conventionally known in the field of organic chemistry.

The reaction time is typically within a range from 20 hours to 30 hours. Furthermore, the reaction time is affected by factors such as the raw materials used (linear or branched), the presence or absence of a catalyst, the esterification temperature, and the amount of excess acid, and therefore may sometimes be shorter than 20 hours or longer than 30 hours. In those cases where a catalyst has been used, the catalyst may be removed by a filtration treatment, adsorption treatment or the like, following completion of the reaction.

The esterification reaction yields a reaction product containing the oligoester according to the present invention. By subjecting this reaction product to a purification treatment using a typical method, the purified oligoester can be obtained. For example, the esterified product may be purified by distillation to remove excess unreacted raw materials. Furthermore, in those cases where it is desirable to improve the hue or the like of the esterified product, a decolorization treatment may be conducted by a typical method to improve the hue.

<Oily Composition>

The oily composition according to the present invention is characterized by containing the oligoester according to the present invention. The oligoester according to the present invention has superior affinity for oily components, and has particularly favorable wax solubility. As a result, the oligoester according to the present invention is suitable as a constituent component of an oily composition, and is particularly ideal as a component to be added to a wax or an oily composition containing a wax.

There are no particular limitations on the form of the oily composition according to the present invention, and the composition may be solid (an oily solid), liquid, or semi-solid. In the case of a semi-solid, the oily composition according to the present invention may have any of various forms, such as jelly-like, gel-like, paste-like, or ointment-like forms. In those cases where the oily composition according to the present invention is an oily solid, there are no particular limitations on the shape of the solid, which may be molded to any of various shapes. Examples of those shapes include rod-like shapes, plate-like shapes, and molded shapes formed by pouring the composition into a dish-like receptacle.

The oligoester according to the present invention included in the oily composition according to the present invention may be composed of one type of the oligoester or at least two types thereof. There are no particular limitations on the amount of the oligoester according to the present invention in the oily composition according to the present invention. However, the amount of the oligoester according to the present invention in an oily solid according to the present invention is preferably within a range from 0.1 to 90% by mass, more preferably from 5 to 70% by mass, and even more preferably from 10 to 70% by mass. When the amount falls within the above-mentioned range, the oily composition exhibits satisfactorily the effects of the oligoester, namely, wax solubility, low-temperature stability, pigment dispersibility, and an improvement in the gloss of the applied surface upon contact with moisture. Moreover, in those cases where the oily composition is an oily solid, the effect of maintaining hardness of the oily solid can also be exhibited satisfactorily.

The oily composition according to the present invention preferably contains a wax that is solid at 25°C in addition to the oligoester according to the present invention. There are no particular limitations on the wax that is solid at 25°C, and examples thereof include hydrocarbon-based waxes such as ozokerite, ceresin, paraffin, petrolatum, Fischer-Tropsch wax, and microcrystalline wax; plant-based waxes such as carnauba wax, candelilla wax, rice wax, sunflower wax, hydrogenated jojoba oil, and Japan wax; animal-based waxes such as beeswax and whale wax; synthetic waxes such as silicone wax, fluorine-based waxes, polyethylene wax, and synthetic beeswax; monoester waxes such as behenyl behenate and stearyl stearate; glyceryl fatty acid esters such as glyceryl tribehenate, glyceryl (behenate/isostearate/eicosadioate), and glyceryl (behenate/eicosadioate); long-chain fatty acids such as stearic acid and behenic acid; and higher alcohols such as stearyl alcohol and behenyl alcohol. The waxes that are solid at 25°C and are contained in the oily composition according to the present invention may be composed of one type of the waxes or at least two types thereof.

There are no particular limitations on the amount of the wax that is solid at 25°C in the oily composition according to the present invention, and the amount may be adjusted appropriately in accordance with the shape required for the oily composition. If the amount of the wax is small, the oily composition becomes liquid, whereas if the amount of the wax is sufficiently large, the oily composition becomes an oily solid. When the oily composition according to the present invention is an oily solid, the amount of the wax relative to the total mass of the solid is preferably within a range from 0.1 to 50% by mass, and more preferably from 1 to 30% by mass. If the amount of the wax is less than 0.1% by mass, satisfactory shape retention cannot be achieved, whereas if the amount exceeds 50% by mass, the resulting oily solid becomes hard, and smooth application may become difficult.

The oily composition according to the present invention may also include one or more other oily components in addition to the oligoester according to the present invention and the wax that is solid at 25°C. These other oily components that may be included in the oily composition according to the present invention may have a form that is liquid, paste-like or solid at 20°C.

Examples of the oily components include hydrocarbons, fatty acid esters, triglycerides, fatty acids, higher alcohols, silicone oils, fluorine-based oils, and derivatives thereof. Specific examples thereof include castor oil, olive oil, avocado oil, palm oil, cacao oil, liquid paraffin, liquid branched paraffin, vaseline, squalane, hydrogenated polyisobutene, hydrogenated polydecene, propane diol di(caprylate/caprate), neopentyl glycol dicaprate, polyglyceryl-6 octacaprylate, glyceryl tri(caprylate/caprate), triethylhexanoin, butyl stearate, ethylhexyl palmitate, coconut alkyl (caprylate/caprate), caprylyl (caprylate/caprate), octyldodecyl myristate, isopropyl myristate, isopropyl lanolin fatty acid ester, hexyl lanolin fatty acid ester, diisopropyl adipate, diisopropyl sebacate, isotridecyl isononanoate, isononyl isononanoate, polyglyceryl decaisostearate, 2-octyldodecanol, diisostearyl malate, polyglyceryl-2 triisostearate, polyglyceryl-2 diisostearate, dipentaerythrityl pentaisostearate, dipentaerythrityl tetraisostearate, pentaerythrityl tetraisostearate, trimethylolpropane triisostearate, dipentaerythrityl hexa(hydroxystearate/stearate/rosinate), cholesteryl hydroxystearate, phytosteryl macadamia nut oil fatty acid ester, dimer dilinoleyl-bis(behenyl/isostearyl/phytosteryl) dimer dilinoleate, di(phytosteryl/octyldodecyl/behenyl) lauroylglutamate, glyceryl (ethylhexanoate/stearate/adipate), oleyl alcohol, dimethylpolysiloxane, methylphenylpolysiloxane, dimethylcyclopolysiloxane, methylhydrogenpolysiloxane, and perfluoropolyether.

There are no particular limitations on the amount of other oily components in the oily composition according to the present invention, and the amount may be adjusted appropriately in accordance with the shape and product quality required for the oily composition. In those cases where the oily composition according to the present invention contains one or more other oily components, the amount of those other components is preferably within a range from 0.1 to 99% by mass, and more preferably from 10 to 90% by mass. Furthermore, those other oily components included in the oily composition according to the present invention may be composed of one type of those other oily components or at least two types thereof.

The oily composition according to the present invention may also include a powder in addition to the oligoester according to the present invention and the wax component that is solid at 25°C. Because the oligoester according to the present invention exhibits favorable pigment dispersibility, when a powder is blended in the oily composition according to the present invention, an oily composition in which the powder is dispersed uniformly throughout the entire composition can be obtained.

Examples of the powder include extender pigments, colored pigments, and pearl pigments.

Examples of the extender pigments include inorganic pigments such as silicic acid, silicic anhydride, magnesium silicate, talc, sericite, mica, kaolin, clay, bentonite, bismuth oxychloride, zirconium oxide, magnesium oxide, zinc oxide, aluminum oxide, calcium sulfate, barium sulfate, magnesium sulfate, calcium carbonate, magnesium carbonate, and composite powders thereof; organic powders composed of polyamide, polyester, polypropylene, polystyrene, polyurethane, nylon, silicone resins, vinyl resins, urea resins, phenol resins, silicone resins, acrylic resins, melamine resins, epoxy resins, polycarbonate resins, divinylbenzene-styrene copolymers, silk powder, cellulose, Nε-lauroyl-L-lysine, metal salts of long-chain alkyl phosphates, N-mono-long chain alkyl acyl basic amino acids, metal soaps, and composite powders thereof; and composite powders of the above-mentioned inorganic powders and organic powders. The particle shape of these powders may be any shape, such as spherical, plate-like, needle-like, granular or amorphous shape.

Examples of the colored pigments include: metal oxides such as titanium oxide, zinc oxide, yellow iron oxide, red iron oxide, black iron oxide, Prussian blue, ultramarine, chromium oxide and chromium hydroxide; metal complexes such as manganese violet and cobalt titanate; inorganic pigments such as carbon black; organic pigments such as tar-based colorants and lake pigments; and natural colorants such as carmine.

Examples of pearl pigments include pearl pigments composed of mica or synthetic phlogopite coated with a colorant such as titanium oxide, iron oxide, silicon oxide, Prussian blue, chromium oxide, carmine, or an organic pigment. These powders may also be subjected to any of various surface treatments such as a water repellency treatment or a water and oil repellency treatment by a typical method.

The powders included in the oily composition according to the present invention may be composed of one type of the powders or at least two types thereof. There are no particular limitations on the amount of the powder included in the oily composition according to the present invention, but for example, the amount is preferably within a range from 1 to 50% by mass relative to the total mass of the oily composition.

The oily composition according to the present invention may also include other components which are different from the wax component that is solid at 25°C, other oily components and powders mentioned above, unless the effects of the oligoester according to the present invention are impaired. Other components may be appropriately selected and used from conventionally-known components typically used as additives in cosmetics, hygiene products or pharmaceutical products.

The oily composition according to the present invention may be produced, for example, by mixing all of the raw materials, including the oligoester according to the present invention, the wax component that is solid at 25°C, other oily components, and, as needed, any other components such as powders, conducting melting under heat if necessary to prepare a uniform mixture, and then molding the mixture into the desired shape. The raw material components may all be mixed together simultaneously, or may be mixed sequentially.

The oily composition according to the present invention may be used, by itself, as a cosmetic, hygiene product, pharmaceutical product, quasi-pharmaceutical product, stationery product or the like. Furthermore, the oily composition may be used as a material to produce these products. Examples of the cosmetic are described below. Examples of the hygiene product include soaps, body shampoos, shampoos, face washes, conditioners, hand creams, body creams, hair creams, hair waxes, and tooth powders. Examples of the pharmaceutical product, quasi-pharmaceutical product or materials thereof include ointments, ointment bases, suppositories, fomentations, creams, and gel pads. Examples of the stationery product include crayons and inks. Among them, a cosmetic, hygiene product, crayon, ink or the like is preferred from the viewpoint that the oligoester according to the present invention exhibits an effect that yields an improvement in the gloss of the applied surface upon contact with moisture, thereby leading to an improvement in the preference.

The oligoester according to the present invention exhibits an effect of maintaining hardness of an oily solid, by adding the oligoester to the oily solid containing a wax that is solid at 25°C. Accordingly, the oligoester according to the present invention can be used as a hardness maintenance agent in an oily solid containing a wax that is solid at 25°C.

The amount of the hardness maintenance agent composed of the oligoester according to the present invention in an oily solid containing a wax that is solid at 25°C is preferably within a range from 0.1 to 90% by mass, more preferably from 5 to 70% by mass, and even more preferably from 10 to 70% by mass.

Furthermore, the oligoester according to the present invention can also be used in a method for maintaining the hardness of an oily solid by incorporating the oligoester in an oily solid containing a wax that is solid at 25°C.

<Cosmetic>

A cosmetic according to the present invention is characterized by containing the oligoester according to the present invention. Cosmetics containing the oligoester according to the present invention exhibit the various actions and effects of the oligoester, and therefore have excellent cosmetic performance and stability.

There are no particular limitations on the form of the cosmetic according to the present invention, and the form may be solid (an oily solid), liquid, or semi-solid. In the case of a semi-solid, the cosmetic according to the present invention may have any of various forms, such as jelly-like, gel-like, paste-like, or ointment-like forms. In those cases where the cosmetic according to the present invention is an oily solid, there are no particular limitations on the shape of the oily solid, and the cosmetic may be molded into any of various shapes. Examples of those shapes include rod-like shapes, plate-like shapes, and molded shapes formed by pouring the composition into a dish-like receptacle.

The oligoester according to the present invention incorporated in the cosmetic according to the present invention may be a single oligoester, or a combination of two or more oligoesters. There are no particular limitations on the amount of the oligoester according to the present invention in the cosmetic according to the present invention. The amount of the oligoester according to the present invention in the cosmetic according to the present invention relative to the total mass of the cosmetic composition, is preferably within a range from 0.1 to 90% by mass, more preferably from 5.0 to 70% by mass, and even more preferably from 10 to 70% by mass. When the amount is within the above-mentioned range, the effects of the oligoester, namely wax solubility, low-temperature stability, pigment dispersibility, and an improvement in the gloss of the applied surface upon contact with moisture, are exhibited satisfactorily in the cosmetic. Moreover, in those cases where the cosmetic is an oily solid, the effect of maintaining hardness of the oily solid can also be exhibited satisfactorily.

The cosmetic according to the present invention preferably contains a wax that is solid at 25°C in addition to the oligoester according to the present invention. There are no particular limitations on the wax that is solid at 25°C, and examples thereof include the same waxes as those exemplified above for inclusion in the aforementioned oily composition. The wax that is solid at 25°C included in the cosmetic according to the present invention may be composed of one type of wax, or two or more types of wax.

There are no particular limitations on the amount of the wax that is solid at 25°C in the cosmetic according to the present invention, and the amount may be adjusted appropriately in accordance with the shape required for the cosmetic. When the cosmetic according to the present invention is an oily solid, the amount of the wax in the solid relative to the total mass of the cosmetic is preferably within a range from 0.1 to 20% by mass, more preferably from 4 to 20% by mass, and even more preferably from 4 to 15% by mass. If the amount of the wax is less than 0.1% by mass, the hardness decreases excessively and satisfactory shape retention cannot be achieved, whereas if the amount exceeds 20% by mass, the resulting oily solid becomes hard, and smooth application may become difficult.

The cosmetic according to the present invention may also include one or more other oily components in addition to the oligoester according to the present invention and the wax that is solid at 25°C. These other oily components that may be included in the cosmetic according to the present invention may have a form that is liquid, paste-like or solid at 20°C. There are no particular limitations on these other oily components, and examples thereof include the same oily components as those exemplified above for inclusion in the aforementioned oily composition. There are no particular limitations on the amount of these other oily components in the cosmetic according to the present invention, and for example, the amount of these other oily components relative to the total mass of the cosmetic is preferably within a range from 0.1 to 99% by mass. The oily component included in the cosmetic according to the present invention may be composed of one type of the oily components or at least two types thereof.

The cosmetic according to the present invention may also include other components besides the oligoester according to the present invention, the wax component that is solid at 25°C, and the above-mentioned other oily components within a range in which the effects exhibited by the combination of the oligoester and the wax component are not impaired. Examples of these other components include powders and surfactants.

The cosmetic according to the present invention may also include a powder in addition to the oligoester according to the present invention and the wax component that is solid at 25°C. Because the oligoester according to the present invention exhibits favorable pigment dispersibility, a cosmetic in which the powder is dispersed uniformly throughout the entire cosmetic can be obtained. There are no particular limitations on the powders that may be included in the cosmetic according to the present invention, and examples thereof include the same powders as those exemplified above for inclusion in the aforementioned oily composition.

There are no particular limitations on the amount of the powder included in the powder according to the present invention, but for example, the amount may be within a range from 1 to 50% by mass relative to the total mass of the cosmetic. The powder included in the cosmetic according to the present invention may be composed of one type of the powders or at least two types thereof.

The cosmetic according to the present invention may also include a surfactant in addition to the oligoester according to the present invention and the wax component that is solid at 25°C. As the surfactant, one or a combination of at least two of nonionic surfactants, anionic surfactants, cationic surfactants, amphoteric surfactants and the like may be included in the cosmetic according to the present invention.

Examples of the nonionic surfactants include monoglycerides, sorbitan fatty acid esters, sucrose fatty acid esters, polyglycerol fatty acid esters, alkanol amides, amine oxides, polyoxyethylene alkyl ethers, polyethylene glycol fatty acid esters, polyoxyethylene sorbitan fatty acid esters, polyoxyethylene glycerol fatty acid esters, polyoxyethylene propylene glycol fatty acid monoesters, polyoxyethylene hydrogenated caster oils, polyoxyethylene fatty acid amides, polyoxyethylene alkylamines, alkyl saccharides, $\alpha$-monoalkyl glyceryl ethers, dimethylpolysiloxanes-polyoxyalkylene copolymers, and dimethylpolysiloxane-monoalkyl glyceryl ether copolymers.

Examples of the anionic surfactants include alkylbenzene sulfonates, alkylnaphthalene sulfonates, polyoxyethylene alkyl ether sulfates, and polyoxyethylene lauryl ether phosphates.

Examples of the cationic surfactants include primary, secondary and tertiary amine salts and quaternary ammonium salts having one or more aliphatic hydrocarbon groups.

Examples of the amphoteric surfactants include sodium $\beta$-laurylaminopropionate, lauryldimethylaminoacetic acid betaine, and 2-alkyl-N-carboxymethyl-N-hydroxyethylimidazolinium betaine.

Examples of other surfactants that may be used include lecithins and soybean saponins. Examples of the lecithins include lecithin, hydrogenated lecithins, lecithin hydroxide, lysolecithin, and hydrogenated lysolecithins. Examples of the hydrogenated lecithins include hydrogenated soybean phospholipids, hydrogenated rapeseed phospholipids, and hydrogenated egg yolk phospholipids.

The cosmetic according to the present invention may also include additives typically added to cosmetics, or water or like, within a range in which the effects achieved by including the oligoester according to the present invention are not impaired. Examples of the additives include antioxidants, antioxidant assistants, preservatives, ultraviolet absorbers, monohydric alcohols, polyhydric alcohols, water-soluble polymers, pH regulators, inorganic salts, salts of organic acids, chelating agents, vitamins, organic solvents, fragrances, various extracts, and ultraviolet scattering agents. One of these additives or a combination of at least two thereof may be used.

Examples of antioxidants, antioxidant assistants, preservatives and ultraviolet absorbers that may be used include the same materials as those described above.

The monohydric alcohols may be lower alcohols or higher alcohols. Examples of the lower alcohols include methanol, ethanol, propanol, isopropanol, isobutyl alcohol, and t-butyl alcohol. Examples of the higher alcohols include cetanol (cetyl alcohol, palmityl alcohol), stearyl alcohol (octadecyl alcohol), isostearyl alcohol (isooctadecanol), oleyl alcohol, cetostearyl alcohol, octyldodecanol, decyltetradecanol, hexyldecanol, behenyl alcohol, lauryl alcohol, lanolin alcohol, and hydrogenated lanolin alcohol. One of the monohydric alcohols may be used alone, or a combination of at least two thereof may be used.

Examples of the polyhydric alcohols include propylene glycol (1,2-propanediol), 1,3-propanediol, 1,3-butylene glycol (1,3-butanediol), pentylene glycol (1,2-pentanediol), neopentylene glycol (2,2-dimethyl-1,3-propanediol), isoprene glycol (3-methyl-1,3-butanediol), dipropylene glycol, glycerol, diglycerol, polyglycerol, polyethylene glycol, pentaerythritol, dipentaerythritol, sorbitol, and sorbitan. One of the polyhydric alcohols may be used alone, or a combination of at least two thereof may be used.

The water-soluble thickeners may be natural water-soluble polymers, semi-synthetic water-soluble polymers, or synthetic water-soluble polymers. One of the water-soluble thickeners may be incorporated in the lip cosmetic according to the present invention alone, or a combination of at least two thereof may be incorporated in the lip cosmetic.

Examples of the natural water-soluble polymers include: plant-based polymers such as agar, glucomannan, gum arabic, tragacanth gum, galactan, guar gum, carob gum, gum karaya, carrageenan, pectin, quince seed (marmelo), algae colloid (brown algae extract) and starches (rice, corn, potato, or wheat); microorganism-based polymers such as xanthan gum, dextran, succinoglycan and pullulan; and animal-based polymers such as collagen, casein, albumin and gelatin.

Examples of the semi-synthetic water-soluble polymers include: starch-based polymers such as carboxymethyl starch and methyl hydroxypropyl starch; cellulose-based polymers such as methyl cellulose, nitrocellulose, methyl hydroxypropyl cellulose, sodium cellulose sulfate, hydroxypropyl cellulose, carboxymethyl cellulose, sodium carboxymethyl cellulose, crystalline cellulose and cellulose powder; and alginate-based polymers such as sodium alginate and propylene glycol alginate.

Examples of the synthetic water-soluble polymers include: vinyl-based polymers such as polyvinyl alcohol, poly(vinyl methyl ether), polyvinylpyrrolidone and carboxyvinyl polymer; polyoxyethylene-based polymers such as polyethylene glycol 20,000, 40,000 and 60,000; polyoxyethylene-polyoxypropylene copolymer-based polymers; acrylic-based polymers such as sodium polyacrylate, poly(ethyl acrylate) and polyacrylamide; polyethyleneimine; and cationic polymers.

Examples of the pH regulators include edetic acid, disodium edetate, citric acid, sodium citrate, sodium hydroxide, potassium hydroxide and triethanolamine. One of the pH regulators may be used alone, or a combination of at least two thereof may be used.

Examples of the inorganic salts include sodium chloride, potassium chloride, magnesium chloride, sodium sulfate, potassium sulfate and magnesium sulfate. Examples of the salts of organic acids include citric acid, malic acid, tartaric acid, and salts thereof, ascorbic acid and salts thereof, and ascorbic acid derivatives and salts thereof.

Examples of the chelating agents include disodium edetate, edetic acid salts, and hydroxyethane diphosphonic acid. One of the chelating agents may be used alone, or a combination of at least two thereof may be used.

Examples of the vitamins include vitamin A, vitamin B1, vitamin B2, vitamin B6, vitamin E, vitamin K, and derivatives thereof, pantothenic acid and derivatives thereof, and biotin.

Examples of the extracts include plant extracts such as aloe vera, witch hazel, hamamelis, cucumber, lemon, lavender and rose.

There are no particular limitations on the cosmetic according to the present invention, and examples thereof include: makeup cosmetics such as lipsticks, foundations, rouges, eyebrow pencils, eye shadows and eye liners; emulsion cosmetics such as milky lotions and creams; liquid cosmetics such as lotions, cleansing creams and cleansing oils; and external skin preparations such as ointments and sunscreens. Among these cosmetics, an oily cosmetic containing a wax that is solid at 25°C is preferred, and an oily solid cosmetic that is an oily solid is particularly preferred in terms of exhibiting satisfactory effects due to the superior wax solubility of the oligoester according to the present invention. Furthermore, cosmetics containing a pigment such as makeup cosmetics are preferred in terms of exhibiting satisfactory effects due to the favorable pigment dispersibility of the oligoester according to the present invention.

Among them, the cosmetic according to the present invention is preferably a lip cosmetic that is applied to the lips such as a lipstick or lip gloss, more preferably a lip cosmetic that contains a wax that is solid at 25°C, and even more preferably an oily solid lip cosmetic that is an oily solid in terms of not only exhibiting satisfactory effects due to the wax solubility and pigment dispersibility, but also exhibiting an effect of the oligoester according to the present invention in improving the gloss of the applied surface upon contact with moisture. The lips are continually exposed to moisture such as saliva and water vapor in the breath, and the lips also frequently make contact with each other, and therefore a lip cosmetic applied to the lips is in a state in which contact with moisture occurs readily. When a lip cosmetic containing the oligoester according to the present invention is applied to lips in such a state, the oligoester makes contact with moisture such as saliva and water vapor in the breath, and therefore the gloss of the applied surface visually improves beyond the gloss level obtained immediately after application.

Accordingly, the oligoester according to the present invention can be used in a method for improving the gloss of the applied surface of a cosmetic by incorporating the oligoester into the cosmetic.

The oligoester according to the present invention can be used particularly favorably in a method for improving the gloss of the applied surface of a lip cosmetic.

The cosmetic according to the present invention can be produced by a typical method, and can be produced in any of various forms, including sticks, pastes, creams, gels and liquids. The form of the cosmetic according to the present invention is preferably an oily solid from the viewpoint of ensuring satisfactory effects of maintaining hardness of the oily solid due to the oligoester according to the present invention. The oligoester according to the present invention may be blended in typical cosmetics without depending on the form thereof.

In those cases where the cosmetic according to the present invention is a lip cosmetic, the oligoester according to the present invention, the wax that is solid at 25°C, and any other components are mixed uniformly while conducting heating, and then the resulting liquid or paste-like mixture is poured into a container having a desired shape, followed by cooling the mixture, for example. Thus, a lip cosmetic having the desired shape can be obtained. The raw material components may all be mixed together simultaneously, or may be mixed sequentially. In those cases where the lip cosmetic is an oily solid, the presence of the oligoester according to the present invention suppresses any decrease in the hardness over time, and allows the shape to be maintained for a longer time period than a lip cosmetic in which no oligoester is blended, thereby providing excellent storage stability. When the cosmetic according to the present invention is in a form in which a wax is blended, favorable storage stability can be achieved not only in a solid form, but also in a semi-solid form (such as a gel-like, paste-like or ointment-like form) or a liquid form. The lip cosmetic may be not only a lipstick, but also a foundation cosmetic, or a finishing gloss-producing cosmetic referred to as a lip gloss, lip color or the like.

In the case of a lip cosmetic, the amount (mass) of the aforementioned other oily components in the cosmetic according to the present invention relative to the total mass of the cosmetic is preferably within a range from 0.1 to 90% by mass, more preferably from 10 to 70% by mass, and even more preferably from 15 to 70% by mass.

[Examples]

The present invention will be described below in further detail with reference to examples. However, needless to say, the scope of the present invention is not limited to these examples. In the following examples, unless stated otherwise, the units "parts" and "%" are "parts by mass" and "% by mass" respectively.

In the tests described below, measurements of the acid value, the saponification value and the hydroxyl value of the esterification reaction products were conducted in accordance with the Japanese Standards of Quasi-drug Ingredients 2006.

[Example 1] Production of Oligoester

A 1 L four-necked flask equipped with a stirrer, a thermometer, a nitrogen gas inlet tube and a water separator was charged with 165.4 g of glycerol, 137.9 g of succinic acid (purity: 99.5%, product name: "succinic acid", manufactured by FUJIFILM Wako Pure Chemical Corporation), 268.0 g of caprylic acid (purity: at least 99% by mass, product name: PALMAC 99-08, manufactured by IOI Acidchem Sdn Bhd) and 178.7 g of capric acid (purity: at least 99% by mass, product name: PALMAC 99-10, manufactured by IOI Acidchem Sdn Bhd), the mixture was heated to 230 to 240°C under a nitrogen gas stream, followed by allowing an esterification reaction to proceed for about 20 hours while removing the produced water. Following completion of the reaction, excess acid was removed to obtain the target esterified product (oligoester).

The obtained oligoester had an acid value of 0.4 mgKOH/g, a saponification value of 447 mgKOH/g, and a hydroxyl value of 8.9 mgKOH/g.

[Examples 2 to 8, and Comparative Examples 1 to 3] Production of Oligoesters

Esterified products of Examples 2 to 8 and Comparative Examples 1 to 3 were obtained in the same manner as the production of the esterified product of Example 1 except that the molar amounts and the blended amounts shown in Tables 1 to 3 were used.

[Table] 1

| Table 1 Blended amounts in esterification reaction | | | | | | | | | |
|---|---|---|---|---|---|---|---|---|---|
| | | Example 1 | | Example 2 | | Example 3 | | Example 4 | |
| Component | Component | mass [g] | molar amount | mass [g] | molar amount | mass [g] | molar amount | mass [g] | molar amount |
| A | Glycerol | 165.4 | 1.00 | 191.9 | 1.00 | 173.4 | 1.00 | 183.7 | 1.00 |
| B | Succinic acid | 137.9 | 0.65 | 172.3 | 0.70 | 166.8 | 0.75 | 188.4 | 0.80 |
| C | Caprylic acid | 268.0 | 1.04 | 291.5 | 0.97 | 245.9 | 0.91 | 256.8 | 0.89 |
| D | Capric acid | 178.7 | 0.58 | 194.3 | 0.54 | 163.9 | 0.51 | 171.1 | 0.50 |
| Mass ratio [(C):(D)] between components (C) and (D) | | 60:40 | | 60:40 | | 60:40 | | 60:40 | |
| Acid value [mgKOH/g] | | 0.4 | | 0.7 | | 1.0 | | 3.6 | |
| Saponification value [mgKOH/g] | | 447 | | 461 | | 467 | | 484 | |
| Hydroxyl value [mgKOH/g] | | 8.9 | | 6.3 | | 1.4 | | 9.4 | |

[Table 2]

| Table 2 Blended amounts in esterification reaction | | | | | | | | | |
|---|---|---|---|---|---|---|---|---|---|
| | | Example 5 | | Example 6 | | Example 7 | | Example 8 | |
| Component | Component | mass [g] | molar amount | mass [g] | molar amount | mass [g] | molar amount | mass [g] | molar amount |
| A | Glycerol | 176.6 | 1.00 | 182.5 | 1.00 | 179.6 | 1.00 | 177.7 | 1.00 |
| B | Succinic acid | 158.6 | 0.70 | 163.8 | 0.70 | 161.2 | 0.70 | 182.3 | 0.80 |
| C | Caprylic acid | 414.9 | 1.50 | 50.3 | 0.18 | 245.5 | 0.87 | 39.0 | 0.14 |
| D | Capric acid | 0 | 0 | 453.4 | 1.33 | 164.7 | 0.49 | 351.0 | 1.06 |
| Mass ratio [(C):(D)] between components (C) and (D) | | 100:0 | | 10:90 | | 59.8:40.2 | | 10:90 | |
| Acid value [mgKOH/g] | | 0.6 | | 1.6 | | 0.5 | | 1.7 | |
| Saponification value [mgKOH/g] | | 482 | | 432 | | 463 | | 452 | |
| Hydroxyl value [mgKOH/g] | | 16.5 | | 7.3 | | 35.0 | | 1.2 | |

[Table 3]

| Table 3 Blended amounts in esterification reaction | | | | | | | |
|---|---|---|---|---|---|---|---|
| | | Comparative Example 1 | | Comparative Example 2 | | Comparative Example 3 | |
| Component | Component | mass [g] | molar amount | mass [g] | molar amount | mass [g] | molar amount |
| A | Glycerol | 179.2 | 1.00 | 199.8 | 1.00 | 180.7 | 1.00 |
| B | Succinic acid | 126.4 | 0.55 | 179.3 | 0.70 | 162.2 | 0.70 |
| C | Caprylic acid | 326.6 | 1.16 | 222.5 | 0.711 | 0 | 0 |
| D | Capric acid | 217.8 | 0.65 | 148.4 | 0.40 | 507.1 | 1.5 |
| Mass ratio [(C):(D)] between components (C) and (D) | | 60:40 | | 60:40 | | 0:100 | |
| Acid value [mgKOH/g] | | 1.1 | | 0.6 | | 0.4 | |
| Saponification value [mgKOH/g] | | 425 | | 469 | | 427 | |
| Hydroxyl value [mgKOH/g] | | 10.9 | | 84 | | 13.4 | |

[Test Example 1] Evaluation of Oligoesters

The wax solubility, improvement in the gloss of the applied surface upon contact with moisture, low-temperature stability, and pigment dispersibility of each oligoester were evaluated using the esterified products obtained in Examples 1 to 8 and Comparative Examples 1 to 3 as test oils. In Comparative Examples 4 to 8, diisostearyl malate, polyglyceryl-2 triisostearate, glyceryl tri(caprylate/caprate), di(phytosteryl/octyldodecyl) lauroylglutamate, and castor oil, which are oils widely used as raw materials of cosmetics, were used as test oils for the purposes of comparison.

In the preparation of the evaluation samples, ceresin wax (product name: CERESIN #810, manufactured by Nikko Rica Corporation), triethylhexanoin (product name: T.I.O, manufactured by The Nisshin OilliO Group, Ltd.), isotridecyl isononanoate (product name: SALACOS 913, manufactured by The Nisshin OilliO Group, Ltd.), hydrogenated polydecene (product name: NOMCORT HP-30, manufactured by The Nisshin OilliO Group, Ltd.), diisostearyl malate (product name: COSMOL 222, manufactured by The Nisshin OilliO Group, Ltd.), polyglyceryl-2 triisostearate (product name: COSMOL 43V, manufactured by The Nisshin OilliO Group, Ltd.), glyceryl tri(caprylate/caprate) (product name: O.D.O, manufactured by The Nisshin OilliO Group, Ltd.), castor oil (product name: Castor Oil Special Grade A, manufactured by Itoh Oil Chemicals Co., Ltd.), and di(phytosteryl/octyldodecyl) lauroylglutamate (product name: ELDEW PS-203, manufactured by Ajinomoto Co., Inc.) were used.

<Evaluation of Wax Solubility>

In an evaluation of a two-component system, 0.5 g of ceresin wax and 4.5 g of the test oil were weighed into a screw-top vial with a volume of 13.5 mL, the mixture was heated to 110°C, and the state of dissolution of the molten mixture was confirmed visually.

In an evaluation of a mixed system, 0.5 g of ceresin wax, 1.5 g of the test oil, 1.65 g of triethylhexanoin, 0.6 g of isotridecyl isononanoate, and 0.75 g of hydrogenated polydecene were weighed into a screw-top vial with a volume of 13.5 mL, the mixture was heated to 110°C, and the state of dissolution of the molten mixture was confirmed visually.

The fact that the evaluation of the two-component system yielded superior wax solubility indicates that better product stability could be achieved upon formation of an oily solid.

The wax solubility of each test oil was evaluated in accordance with the following evaluation criteria. An evaluation of a or b was assessed to indicate superior wax solubility.

Evaluation Criteria of Wax Solubility:

    a: A transparent solution was obtained in the two-component system.
    b: A transparent solution was obtained in the mixed system.
    c: A transparent solution was not obtained.

<Evaluation Test of Improvement in Gloss of the Applied Surface upon Contact with Moisture>

The effect of improvement in the gloss of the surface applied with the test oil upon contact with moisture was evaluated by five specialist panelists in the following manner.

First, 30 μL of the test oil was applied thinly to the inside of the forearm with the index finger, the applied surface was exposed to running water at 30°C while being rubbed lightly back and forth 20 times with the index finger, and the applied surface was then removed from the running water. The applied surface was shaken to remove water droplets, and then left to stand for five minutes to dry. An evaluation was then conducted by comparing the gloss of the applied surface following drying with the gloss of the applied surface immediately following application. The effect of each test oil in improving the gloss of the applied surface upon contact with moisture was evaluated in accordance with the following evaluation criteria, and an evaluation of a or b was assessed to indicate improved gloss.

Evaluation Criteria of Improvement in Gloss of the Applied Surface upon Contact with Moisture:

    5: The gloss improved from the gloss immediately following application.
    4: The gloss improved slightly from the gloss immediately following application.
    3: No change in gloss from the gloss immediately following application.
    2: The gloss decreased slightly from the gloss immediately following application.
    1: The gloss decreased from the gloss immediately following application.

Evaluation of Improvement in Gloss of the Applied Surface upon Contact with Moisture:

    a: Evaluation value (average) was at least 4.0 but not more than 5.0.
    b: Evaluation value (average) was at least 3.5 but less than 4.0.
    c: Evaluation value (average) was at least 2.5 but less than 3.5.
    d: Evaluation value (average) was at least 1.0 but less than 2.5.

<Evaluation of Low-Temperature Stability>

First, 10 g of each test oil was placed in a screw-top vial with a volume of 13.5 mL, the vial was stored for one week in an environment at 0°C, and the presence or absence of crystal precipitation in the oil was then inspected visually.

The low-temperature stability of each test oil was evaluated in accordance with the following evaluation criteria. An evaluation of a or b was assessed to indicate favorable low-temperature stability.

Evaluation Criteria of Low-Temperature Stability:

   a: A transparent liquid was obtained.
   b: Minor crystal precipitation was observed.
   c: Obvious crystal precipitation was observed.

<Evaluation of Pigment Dispersibility>

First, 4 g of the test oil was mixed with 20 g of titanium oxide (product name: TIPAQUE A-100, manufactured by Ishihara Sangyo Kaisha, Ltd.), and then octyl palmitate (product name: SALACOS P-8, manufactured by The Nisshin OilliO Group, Ltd.) was added thereto gradually, followed by measuring the amounts of octyl palmitate added until the wet-point where the entire mixture became a single mixture and the flow point where the entire mixture began to flow when inclined to determine the wet-point value and the flow point value, respectively.

A lower wet-point value, and a smaller difference between the wet-point value and the flow point value indicate superior pigment dispersibility of the test oil.

The pigment dispersibility of each test oil was evaluated in accordance with the following evaluation criteria. An evaluation of a or b was assessed to indicate superior pigment dispersibility.

Evaluation Criteria of Pigment Dispersibility:

   a: The wet-point value was less than 2.0 g, and the difference ([flow point value] - [wet-point value]) was less than 3.0 g.

   b: The wet-point value was less than 2.0 g, and the difference ([flow point value] - [wet-point value]) was at least 3.0 g but less than 20.0 g.

   c: The wetting point value was 2.0 g or higher, and the difference ([flow point value] - [wetting point value]) was less than 20.0 g.

   d: The wetting point value was 2.0 g or higher, and the difference ([flow point value] - [wet-point value]) was 20.0 g or higher.

[Table 4]

| Table 4 Evaluation results of each compound | | | | | |
|---|---|---|---|---|---|
| | Example 1 | Example 2 | Example 3 | Example 4 | Example 5 |
| Evaluated compound | Oligoester of Example 1 | Oligoester of Example 2 | Oligoester of Example 3 | Oligoester of Example 4 | Oligoester of Example 5 |
| Wax solubility | a | a | a | b | b |
| Gloss improvement | 3.6 | 4.2 | 3.8 | 4.0 | 4.4 |
| Gloss improvement evaluation | b | a | b | a | a |
| Low-temperature stability | a | a | a | a | a |
| Pigment dispersibility (wet-point [g]) | 2.0 | 1.9 | 1.9 | 1.8 | 1.8 |
| Pigment dispersibility (flow point - wet-point [g]) | 2.4 | 0.6 | 0.4 | 2.7 | 0.2 |

(continued)

| Table 4 Evaluation results of each compound | | | | | |
|---|---|---|---|---|---|
| | Example 1 | Example 2 | Example 3 | Example 4 | Example 5 |
| Evaluated compound | Oligoester of Example 1 | Oligoester of Example 2 | Oligoester of Example 3 | Oligoester of Example 4 | Oligoester of Example 5 |
| Pigment dispersibility evaluation | a | a | a | a | a |

[Table 5]

| Table 5 Evaluation results of each compound | | | | | | |
|---|---|---|---|---|---|---|
| | Example 6 | Example 7 | Example 8 | Comparative Example 1 | Comparative Example 2 | Comparative Example 3 |
| Evaluated compound | Oligoester of Example 6 | Oligoester of Example 7 | Oligoester of Example 8 | Oligoester of Comparative Example 1 | Oligoester of Comparative Example 2 | Oligoester of Comparative Example 3 |
| Wax solubility | a | b | a | a | c | a |
| Gloss improvement | 3.8 | 3.6 | 3.6 | 2.4 | 3.3 | 3.6 |
| Gloss improvement evaluation | b | b | b | d | c | b |
| Low-temperature stability | b | a | b | a | a | c |
| Pigment dispersibility (wet-point [g]) | 1.7 | 1.8 | 1.6 | 1.9 | 1.9 | 1.7 |
| Pigment dispersibility (flow point - wet-point [g]) | 0.8 | 0.4 | 1.1 | 3.4 | 0.7 | 1.8 |
| Pigment dispersibility evaluation | a | a | a | b | a | a |

[Table 6]

| Table 6 Evaluation results of each compound | | | | | |
|---|---|---|---|---|---|
| | Comparative Example 4 | Comparative Example 5 | Comparative Example 6 | Comparative Example 7 | Comparative Example 8 |
| Evaluated compound | Diisostearyl malate | Polyglyceryl-2 triisostearate | Glyceryl tri(caprylate/ caprate) | Di(phytosteryl/ octyldodecyl) lauroyl glutamate | Castor oil |
| Wax solubility | a | a | a | a | a |
| Gloss improvement | 2.6 | 3.0 | 2.0 | 2.0 | 2.4 |
| Gloss improvement evaluation | c | c | d | d | d |
| Low-temperature stability | a | a | a | a | a |
| Pigment dispersibility (wet-point [g]) | 3.5 | 1.0 | 7.7 | 1.8 | 7.0 |

(continued)

| Table 6 Evaluation results of each compound | | | | | |
|---|---|---|---|---|---|
| | Comparative Example 4 | Comparative Example 5 | Comparative Example 6 | Comparative Example 7 | Comparative Example 8 |
| Evaluated compound | Diisostearyl malate | Polyglyceryl-2 triisostearate | Glyceryl tri(caprylate/ caprate) | Di(phytosteryl/ octyldodecyl) lauroyl glutamate | Castor oil |
| Pigment dispersibility (flow point - wet-point [g]) | 28.5 | 10.0 | 40.2 | 13.5 | 50.0 |
| Pigment dispersibility evaluation | d | b | d | b | d |

<Evaluation Results of Oligoesters>

As is evident from the results shown in Tables 4 to 6, the oligoesters according to the present invention of Examples 1 to 8 exhibited superior wax solubility, improvement in the gloss of the applied surface upon contact with moisture and low-temperature stability, and also exhibited favorable pigment dispersibility. In contrast, it was evident that the oligoester of Comparative Example 1n exhibited o effect in improving the gloss of the applied surface upon contact with moisture, the oligoester of Comparative Example 2 exhibited poor wax solubility, and the oligoester of Comparative Example 3 exhibited poor low-temperature stability. Based on the results of Comparative Example 3, it was surmised that the low-temperature stability of oligoesters synthesized using only a linear fatty acid having 10 or more carbon atoms deteriorated. The oils of Comparative Examples 4 to 8, commonly used as raw materials of cosmetics, exhibited no effect in improving the gloss of the applied surface upon contact with moisture. Furthermore, the oils of Comparative Examples 4, 6 and 8 exhibited inferior pigment dispersibility. Moreover, the castor oil of Comparative Example 8 was a natural product, and therefore there were some problems in terms of stability of the product quality.

[Examples 9 to 16 and Comparative Examples 9 to 15] Oily Solids

Oily solids were produced using the formulations shown in Tables 7 to 9, and the initial hardness and the hardness maintenance rate following cycle testing were evaluated. The unit "%" in the raw material compositions shown in Tables 7 to 9 indicates "% by mass".

<Production of Oily Solids>

All of raw material components shown in Tables 7 to 9were stirred and mixed under a heated state at 100°C, and the resulting mixture was then poured into a polycarbonate container (having a diameter of 38 mm and a depth of 17 mm) and cooled to room temperature to produce a circular cylindrical oily solid.

<Evaluation of Hardness>

The maximum stress (gf) when a spherical plunger having a diameter of 5 mm was forced into the oily solid inside the polycarbonate container to the depth of 2.5 mm was measured as the hardness with a gel hardness meter (product name: Compact Tabletop Tester EZ-Test EZ-SX, manufactured by Shimadzu Corporation).

<Evaluation of Hardness Maintenance Rate>

Each produced oily solid was subjected to a measurement of the hardness following standing for 24 hours in a constant temperature chamber at 25°C (the initial hardness) and the hardness following conducting a cycle storage test in which steps of storing the oily solid at 5°C for 12 hours and then holding the oily solid at 40°C for 12 hours were repeated for two weeks (the post-storage test hardness) to determine the hardness maintenance rate (%) of the post-storage test hardness relative to 100% of the initial hardness.
The hardness maintenance rate of each oily solid was evaluated in accordance with the following evaluation criteria. An evaluation of a or b was assessed to indicate a superior hardness maintenance rate.

Evaluation Criteria of Hardness Maintenance Rate:

a: 80% or higher

b: At least 70% but less than 80%

c: Less than 70%

[Table 7]

| Table 7 Evaluation results of oily solids | | | | | |
|---|---|---|---|---|---|
| Raw materials | Example 9 | Example 10 | Example 11 | Example 12 | Example 13 |
| Oligoester of Example 1 [%] | 30 | - | - | - | - |
| Oligoester of Example 2 [%] | - | 30 | - | - | - |
| Oligoester of Example 3 [%] | - | - | 30 | - | - |
| Oligoester of Example 4 [%] | - | - | - | 30 | - |
| Oligoester of Example 5 [%] | - | - | - | - | 30 |
| Ceresin [%] | 15 | 15 | 15 | 15 | 15 |
| Triethylhexanoin [%] | 30.2 | 30.2 | 30.2 | 30.2 | 30.2 |
| Isotridecyl isononanoate [%] | 11 | 11 | 11 | 11 | 11 |
| Hydrogenated polydecene [%] | 13.8 | 13.8 | 13.8 | 13.8 | 13.8 |
| Total [%] | 100 | 100 | 100 | 100 | 100 |
| Hardness maintenance rate [%] | 91 | 82 | 80 | 74 | 85 |
| Hardness maintenance rate evaluation | a | a | a | b | a |

[Table 8]

| Table 8 Evaluation results of oily solids | | | | | |
|---|---|---|---|---|---|
| Raw materials | Example 14 | Example 15 | Example 16 | Comparative Example 9 | Comparative Example 10 |
| Oligoester of Example 6 [%] | 30 | - | - | - | - |
| Oligoester of Example 7 [%] | - | 30 | - | - | - |
| Oligoester of Example 8 [%] | - | - | 30 | - | - |
| Oligoester of Comparative Example 1 [%] | - | - | - | 30 | - |
| Oligoester of Comparative Example 3 [%] | - | - | - | - | 30 |
| Ceresin [%] | 15 | 15 | 15 | 15 | 15 |
| Triethylhexanoin [%] | 30.2 | 30.2 | 30.2 | 30.2 | 30.2 |
| Isotridecyl isononanoate [%] | 11 | 11 | 11 | 11 | 11 |
| Hydrogenated polydecene [%] | 13.8 | 13.8 | 13.8 | 13.8 | 13.8 |
| Total [%] | 100 | 100 | 100 | 100 | 100 |
| Hardness maintenance rate [%] | 80 | 85 | 71 | 62 | 84 |
| Hardness maintenance rate evaluation | a | a | b | c | a |

[Table 9]

| Table 9 Evaluation results of oily solids | | | | | |
|---|---|---|---|---|---|
| Raw materials | Comparative Example 11 | Comparative Example 12 | Comparative Example 13 | Comparative Example 14 | Comparative Example 15 |
| Oligoester of Comparative Example 2 [%] | 30 | - | - | - | - |
| Diisostearyl malate [%] | - | 30 | - | - | - |
| Polyglyceryl-2 triisostearate [%] | - | - | 30 | - | - |
| Glyceryl tri(caprylate/caprate) [%] | - | - | - | 30 | - |
| Di(phytosteryl/octyldodecyl) lauroylglutamate [%] | - | - | - | - | 30 |
| Ceresin [%] | 15 | 15 | 15 | 15 | 15 |
| Triethylhexanoin [%] | 30.2 | 30.2 | 30.2 | 30.2 | 30.2 |
| Isotridecyl isononanoate [%] | 11 | 11 | 11 | 11 | 11 |
| Hydrogenated polydecene [%] | 13.8 | 13.8 | 13.8 | 13.8 | 13.8 |
| Total [%] | 100 | 100 | 100 | 100 | 100 |
| Hardness maintenance rate [%] | 61 | 65 | 64 | 68 | 64 |
| Hardness maintenance rate evaluation | c | c | c | c | c |

<Evaluation Results of Oily Solids>

As is evident from the results shown in Tables 7 to 9, the oily solids containing the oligoester according to the present invention exhibited favorable hardness maintenance rates. Although the oily solid of Comparative Example 10 exhibited a favorable hardness maintenance rate, the oligoester of Comparative Example 3 contained in this oily solid exhibited poor low-temperature stability. The oily solids of Comparative Examples 9 and 11 to 15 exhibited poor hardness maintenance rates.

[Examples 17 and 18 and Comparative Examples 16 to 18] Lipsticks (Stick-type)

Lipsticks (stick-type) were produced using the formulations shown in Tables 10 and 11, and the storage stability, the pigment dispersibility, and the sensation upon use were evaluated. The unit "%" in Tables 10 and 11 indicates "% by mass".

< Raw Materials of Lipstick (stick-type)>

As the raw materials of the lipsticks (stick-type) shown in Tables 10 and 11, a synthetic wax (product name: Lip Wax A-4, manufactured by Japan Natural Products Co., Ltd.), a mixture of a synthetic wax and an ethylene/propylene copolymer (product name: Lip Wax PZ80-20, manufactured by Japan Natural Products Co., Ltd.), microcrystalline wax (product name: Multiwax W445, manufactured by Sonneborn, LLC), vaseline (product name: NOMCORT W, manufactured by The Nisshin OilliO Group, Ltd.), di(octyldodecyl/phytosteryl/behenyl) lauroylglutamate (product name: ELDEW PS-304, manufactured by Ajinomoto Co., Inc.), lanolin (product name: Ecolano LN-E, manufactured by Nippon Fine Chemical Co., Ltd.), hydrogenated polyisobutene (product name: PARLEAM 18, manufactured by NOF Corporation), polyglyceryl-2 diisostearate (product name: COSMOL 42V, manufactured by The Nisshin OilliO Group, Ltd.), and titanium oxide (product name: TIPAQUE CR-50, manufactured by Ishihara Sangyo Kaisha, Ltd.) were used. As the di(phytosteryl/octyldodecyl) lauroylglutamate, diisostearyl malate, triethylhexanoin, isotridecyl isononanoate, hydrogenated polydecene and ceresin wax, the same materials as those used in Test Example 1 were used.

<Production of Lipstick (stick-type)>

The colorants of section C were mixed thoroughly and then dispersed with a roll mill. The components of sections A to C were stirred and mixed under a heated state at 100°C to obtain a uniform mixture, followed by degassing the mixture under reduced pressure, pouring some of the mixture into a bullet-shaped lipstick mold and then cooling the resultant to room temperature to obtain a lipstick (stick-type). At the same time, some of the mixture was poured into a polycarbonate container (having a diameter of 38 mm and a depth of 17 mm) and cooled to room temperature to produce a circular cylindrical lipstick. The lipstick (stick-type) was used to evaluate the sensation upon use. The circular cylindrical lipstick was used to measure the hardness and evaluate the pigment dispersibility.

<Evaluation of Hardness>

The maximum stress (g) when a spherical plunger having a diameter of 5 mm was forced into the lipstick inside the polycarbonate container to the depth of 2.5 mm was measured as the hardness with a gel hardness meter (product name: SD700, manufactured by Sun Scientific Co., Ltd.).

<Evaluation of Storage Stability>

The storage stability of each lipstick was evaluated using the change in hardness as an indicator. Specifically, the hardness maintenance rate (%) was measured in the same manner as that described above for the oily solids, and the storage stability was evaluated in accordance with the following evaluation criteria. A higher hardness maintenance rate was evaluated as indicating superior storage stability.
The storage stability of each lipstick was evaluated in accordance with the following evaluation criteria. An evaluation of a or b was assessed to indicate superior storage stability.

Evaluation Criteria of Storage Stability:

   a: The hardness maintenance rate was 85% or higher.
   b: The hardness maintenance rate was at least 75% but less than 85%.
   c: The hardness maintenance rate was less than 75%.

<Evaluation of External Coloration>

First, a color difference meter (product name: Color Reader CR-10, manufactured by Konika Minolta, Inc.) was used to measure the color of the above lipstick inside the transparent polycarbonate container. Subsequently, the lipstick was removed from the container, kneaded thoroughly on top of a glass plate using a spatula, and then once again poured into a transparent polycarbonate container to measure the color by the same method as described above. The color difference (ΔE) in the L*a*b* color space between before kneading and after kneading was determined, and a smaller color difference was deemed to indicate a smaller change between the external color and the applied color, and therefore better external coloration.

[Numerical formula 1]

$$\Delta E = \sqrt{(\Delta L*)^2 + (\Delta a*)^2 + (\Delta b*)^2}$$

<Evaluation of Sensation upon Use>

Each lipstick was evaluated by five specialist panelists in terms of evaluation items such as "easiness in adhesion", "easiness in spread", "absence of color irregularity" and "low stickiness".
The sensation upon use of each lipstick was evaluated in accordance with the following evaluation criteria. An evaluation of a or b was assessed to indicate favorable sensation upon use.

Evaluation Criteria of Sensation upon Use:

5: Good
4: Somewhat good
3: Typical
2: Slightly poor
1: Poor

Evaluation of Sensation upon Use:

a: The evaluation value (average) was at least 4.0 but not more than 5.0.
b: The evaluation value (average) was at least 3.5 but less than 4.0.
c: The evaluation value (average) was at least 2.5 but less than 3.5.
d: The evaluation value (average) was at least 1.0 but less than 2.5.

[Table 10]

| Table 10 Evaluation results of Lipstick (stick-type) | | | | | |
|---|---|---|---|---|---|
| Section | Component | Example 17 | Comparative Example 16 | Comparative Example 17 |
| A | Synthetic wax [%] | 7 | 7 | 7 |
| | Mixture of synthetic wax and ethylene/propylene copolymer [%] | 2 | 2 | 2 |
| | Microcrystalline wax [%] | 2 | 2 | 2 |
| B | Vaseline [%] | 2 | 2 | 2 |
| | Di(octyldodecyl/phytosteryl/behenyl) | 1 | 1 | 1 |
| | lauroylglutamate [%] | | | |
| | Oligoester of Example 2 [%] | 15 | - | - |
| | Oligoester of Comparative Example 1 [%] | - | 15 | - |
| | Di(phytosteryl/octyldodecyl) lauroylglutamate [%] | - | - | 15 |
| | Diisostearyl malate [%] | 17 | 17 | 17 |
| | Triethylhexanoin [%] | 18 | 18 | 18 |
| | Isotridecyl isononanoate [%] | 12 | 12 | 12 |
| | Hydrogenated polydecene [%] | 16 | 16 | 16 |
| C | Diisostearyl malate [%] | 3 | 3 | 3 |
| | Polyglyceryl-2 diisostearate [%] | 2 | 2 | 2 |
| | Red No. 202 [%] | 0.5 | 0.5 | 0.5 |
| | Yellow No. 4 aluminum lake [%] | 0.3 | 0.3 | 0.3 |
| | Red iron oxide [%] | 0.6 | 0.6 | 0.6 |
| | Titanium oxide [%] | 1.6 | 1.6 | 1.6 |
| Total [%] | | 100 | 100 | 100 |
| Hardness maintenance rate [%] | | 87 | 81 | 77 |
| Storage stability | | a | b | b |

(continued)

| Table 10 Evaluation results of Lipstick (stick-type) | | Example 17 | Comparative Example 16 | Comparative Example 17 |
|---|---|---|---|---|
| Section | Component | | | |
| External Coloration (ΔE) | | 0.67 | 1.90 | 1.90 |
| Easiness in adhesion | | a | d | b |
| Easiness in spread | | a | b | d |
| Absence of color irregularity | | a | b | d |

[Table 11]

| Table 11 Evaluation results of Lipstick (stick-type) | | Example 18 | Comparative Example 18 |
|---|---|---|---|
| Section | Component | | |
| A | Ceresin [%] | 10 | 10 |
| B | Lanolin [%] | 12 | 12 |
| | Vaseline [%] | 5 | 5 |
| | Oligoester of Example 2 [%] | 25 | - |
| | Diisostearyl malate [%] | - | 25 |
| | Triethylhexanoin [%] | 15 | 15 |
| | Isotridecyl isononanoate [%] | 10 | 10 |
| | Hydrogenated polydecene [%] | 9 | 9 |
| | Hydrogenated polyisobutene [%] | 6 | 6 |
| C | Diisostearyl malate [%] | 5 | 5 |
| | Red No. 202 [%] | 0.5 | 0.5 |
| | Yellow No. 4 aluminum lake [%] | 0.3 | 0.3 |
| | Red iron oxide [%] | 0.6 | 0.6 |
| | Titanium oxide [%] | 1.6 | 1.6 |
| Total [%] | | 100 | 100 |
| Hardness maintenance rate [%] | | 90 | 71 |
| Storage stability | | a | c |
| Easiness in adhesion | | b | b |
| Easiness in spread | | a | d |
| Low stickiness | | a | c |

<Evaluation Results of Lipsticks (stick-type)>

As is evident from the results shown in Tables 10 and 11, the lipstick of Example 17 had favorable storage stability as a stick-type cosmetic, exhibited excellent external coloration, displayed good sensation upon use, such as easiness in adhesion and easiness in spread, and was able to be applied without color irregularities. The lipstick of Example 18 also had favorable storage stability, and exhibited good sensation upon use, such as easiness in adhesion, easiness in spread, and low stickiness. Although lipsticks sometimes suffer from a problem in which repeated temperature variations cause a decrease in hardness, leading to breakage of the stick during use, it was confirmed that the cosmetic containing the oligoester according to the present invention was less likely to cause decrease in the hardness during storage, and there was little concern.

[Example 19] Lip Gloss

A lip gloss was produced using the formulation shown in Table 12. The unit "%" in Table 12 indicates "% by mass".

The obtained lip gloss had a paste-like form, were able to be applied smoothly during application, yielded a coating film with satisfactory thickness, yielded improved gloss following application, displayed good retention of the gloss, and had favorable storage stability. It was assumed that the improvement in the gloss following application was due to moisture in the saliva or breath coming into contact with the applied surface.

<Raw Materials of Lip Gloss>

As raw materials of the lip gloss shown in Table 12, dipentaerythrityl pentaisostearate (product name: SALACOS DP-518N, manufactured by The Nisshin OilliO Group, Ltd.), pentaerythrityl tetraisostearate (product name: SALACOS 5418V, manufactured by The Nisshin OilliO Group, Ltd.), and glyceryl (behenate/eicosadioate) (product name: NOMCORT HK-G, manufactured by The Nisshin OilliO Group, Ltd.) were used.

<Production of Lip Gloss>

The components of section A were stirred and mixed under a heated state at 100°C to obtain a uniform mixture, followed by degassing the mixture under reduced pressure and then cooling the resultant to room temperature to obtain a lip gloss..

[Table 12]

| Table 12 Formulation of lip gloss | | |
|---|---|---|
| Section | Component | Example 19 |
| A | Oligoester of Example 2 [%] | 40 |
| | Dipentaerythrityl pentaisostearate [%] | 50 |
| | Pentaerythrityl tetraisostearate [%] | 8 |
| | Glyceryl (behenate/eicosadioate) [%] | 2 |
| Total [%] | | 100 |

[Example 20 and Comparative Example 19] Cleansing Oils

Cleansing oils were produced using the formulations shown in Table 13, and the sensation upon use was evaluated. The unit "%" in Table 13 indicates "% by mass".

<Raw Materials of Cleansing Oil>

As raw materials of the cleansing oils shown in Table 13, PEG-20 glyceryl triisostearate (product name: SALACOS GE-318, manufactured by The Nisshin OilliO Group, Ltd.) and cetyl ethylhexanoate (product name: SALACOS 816T, manufactured by The Nisshin OilliO Group, Ltd.) were used. The glyceryl (behenate/eicosadioate) was the same as that used in Example 19.

<Production of Cleansing Oils>

The components of section A were stirred and mixed under a heated state at 80°C to obtain uniform mixtures, followed by cooling the mixtures to room temperature while conducting stirring to obtain cleansing oils.

<Evaluation of Storage Stability>

Each cleansing oil was evaluated in terms of storage stability using the external appearance upon storage at 50°C for one month as an indicator. Specifically, the cleansing oil was placed in a screw-top vial with a volume of 30 mL and the presence or absence of a separated transparent layer on top of the cleansing oil was inspected to evaluate the storage stability in accordance with the following evaluation criteria.

Evaluation Criteria of Storage Stability:

a: No separated layer was observed and the storage stability was favorable.
b: A distinct separated layer was observed and the storage stability was poor.

<Evaluation of Sensation upon Use>

Each cleansing oil was evaluated by five specialist panelists in terms of the evaluation items such as "frictional feeling during application", "makeup removability" and "rinsability".
The sensation upon use of each cleansing oil was evaluated in accordance with the following evaluation criteria. An evaluation of a or b was assessed to indicate favorable sensation upon use.

Evaluation Criteria of Sensation upon Use:

5: Good
4: Somewhat good
3: Typical
2: Slightly poor
1: Poor

Evaluation of Sensation upon Use:

a: The evaluation value (average) was at least 4.0 but not more than 5.0.
b: The evaluation value (average) was at least 3.5 but less than 4.0.
c: The evaluation value (average) was at least 2.5 but less than 3.5.
d: The evaluation value (average) was at least 1.0 but less than 2.5.

[Table 13]

| Table 13 Evaluation results of cleansing oils | | | |
|---|---|---|---|
| Section | Component | Example 20 | Comparative Example 19 |
| A | PEG-20 glyceryl triisostearate [%] | 20 | 20 |
| | Cetyl ethylhexanoate [%] | 59 | 79 |
| | Oligoester of Example 2 | 20 | - |
| | Glyceryl (behenate/eicosadioate) [%] | 1 | 1 |
| Total [%] | | 100 | 100 |
| Storage stability | | a | b |
| Frictional feeling during application | | a | c |
| Makeup removability | | b | b |
| Rinsability | | a | d |

<Evaluation Results of Cleansing Oils>

As is evident from the results shown in Table 13, the cleansing oil of Example 20 had favorable storage stability as a cleansing oil, produced no frictional feeling during application, and exhibited excellent makeup removability. This ability to be compatible with makeup without causing any frictional feeling is important in terms of not imparting stimulus to the skin, and is advantageous from the perspective of skin care. The cleansing oil could be easily removed with running water after making the cleansing oil mix with the makeup, and therefore it was confirmed that the usability of the cleansing oil was favorable.

[Example 21] Water-in-Oil Foundation

A water-in-oil foundation was produced using the formulation shown in Table 14. The unit "%" in Table 14 indicates "% by mass".

< Raw Materials of Water-in-Oil Foundation>

As raw materials of the water-in-oil foundation shown in Table 13, BG (product name: 1,3-butylene glycol, manufactured by Daicel Corporation), glycerol (product name: concentrated glycerol for cosmetics, manufactured by Sakamoto Yakuhin Kogyo Co., Ltd.), dipentaerythrityl tripolyhydroxystearate (product name: SALACOS WO-6, manufactured by The Nisshin OilliO Group, Ltd.), dimethicone (product name: KF-96A-6cs, manufactured by Shin-Etsu Chemical Co., Ltd.), ethylhexyl methoxycinnamate (product name: NOMCORT TAB, manufactured by The Nisshin OilliO Group, Ltd.), polyhydroxystearic acid (product name: SALACOS HS-6C, manufactured by The Nisshin OilliO Group, Ltd.), disteardimonium hectorite (product name: Bentone 38V, manufactured by Elementis pic), cetyl dimethicone copolyol (product name: ABIL EM 90, manufactured by Evonik Operations GmbH), PEG-10 dimethicone (product name: KF-6017, manufactured by Shin-Etsu Chemical Co., Ltd.), cetanol (product name: KALCOL 6870, manufactured by Kao Corporation), behenyl alcohol (product name: KALCOL 220-80, manufactured by Kao Corporation), microparticulate titanium oxide dispersion (product name: Cosmeserve WP-UF(V), manufactured by Dainihonkasei Co., Ltd.), microparticulate zinc oxide dispersion (product name: Cosmeserve WPA-STD(V)-2, manufactured by Dainihonkasei Co., Ltd.), (dimethicone/methicone) copolymer-treated powder (manufactured by Daito Kasei Kogyo Co., Ltd.), polymethyl methacrylate (product name: Matsumoto Microsphere M-100, manufactured by Matsumoto Yushi-Seiyaku Co., Ltd.), and tocopherol (product name: Tocopherol 100, manufactured by The Nisshin OilliO Group, Ltd.) were used. The triethylhexanoin, cetyl ethylhexanoate and hydrogenated polydecene were the same as those used in Test Example 1. The titanium oxide was the same as that used in Example 17.

<Production of Water-in-Oil Foundation>

The components of sections A and B were stirred and mixed separately under a heated state at 70°C, the mixture of section A was added to the mixture of section B, and then the resulting mixture was emulsified and dispersed with a homo mixer, followed by cooling the resultant to obtain a water-in-oil foundation.

[Table 14]

| Table 14 Formulation of water-in-oil foundation | | |
|---|---|---|
| Section | Component | Example 21 |
| A | Water [%] | 20.75 |
| | BG [%] | 5 |
| | Glycerol [%] | 4 |
| | Na chloride [%] | 1 |
| | Methylparaben [%] | 0.1 |

(continued)

| Section | Component | Example 21 |
|---|---|---|
| Table 14 Formulation of water-in-oil foundation | | |
| B | Oligoester of Example 2 [%] | 5 |
| | Dipentaerythrityl tripolyhydroxystearate [%] | 0.5 |
| | Triethylhexanoin [%] | 5 |
| | Dimethicone [%] | 4 |
| | Ethylhexyl methoxycinnamate [%] | 5 |
| | Cetyl ethylhexanoate [%] | 3 |
| | Hydrogenated polydecene [%] | 5 |
| | Polyhydroxystearic acid [%] | 0.3 |
| | Disteardimonium hectorite [%] | 2 |
| | Cetyl dimethicone copolyol [%] | 3 |
| | PEG-10 dimethicone [%] | 2 |
| | Cetanol [%] | 3.5 |
| | Behenyl alcohol [%] | 2 |
| | Microparticulate titanium oxide dispersion [%] | 9 |
| | Microparticulate zinc oxide dispersion [%] | 8 |
| | Titanium oxide [%] | 3 |
| | (Dimethicone/methicone) copolymer-treated talc [%] | 5 |
| | Polymethyl methacrylate [%] | 2 |
| | (Dimethicone/methicone) copolymer-treated iron oxide (red) [%] | 0.26 |
| | (Dimethicone/methicone) copolymer-treated iron oxide (black) [%] | 0.24 |
| | (Dimethicone/methicone) copolymer-treated iron oxide (yellow) [%] | 1.2 |
| | Propylparaben [%] | 0.05 |
| | Tocopherol [%] | 0.1 |
| Total [%] | | 100 |

<Evaluation Results of Water-in-Oil Foundation>

The water-in-oil foundation of Example 21 had a cream-like form, was able to be applied smoothly during application, yielded a uniform coating film with no color irregularities, produced a beautiful glossy finish, and exhibited a favorable moisturizing sensation.

[Example 22] Hair Oil

A hair oil was produced using the formulation shown in Table 15. The unit "%" in Table 15 indicates "% by mass".

<Raw Materials of Hair Oil>

As raw materials of the hair oil shown in Table 15, neopentyl glycol dicaprate (product name: ESTEMOL N-01, manufactured by The Nisshin OilliO Group, Ltd.), dimethicone (product name: KF-96A-10cs, manufactured by Shin-Etsu Chemical Co., Ltd.), phytosteryl oleate (product name: SALACOS PO, manufactured by The Nisshin OilliO Group, Ltd.), and isododecane (product name: MARUKAZOL R, manufactured by Maruzen Petrochemical Co., Ltd.) were used. The dipentaerythrityl tripolyhydroxystearate and tocopherol were the same as those used in Example 21.

<Production of Hair Oil>

The components of section A were stirred and mixed at room temperature to form a uniform transparent solution, thus obtaining a hair oil.

[Table 15]

| Table 15 Formulation of hair oil | | |
|---|---|---|
| Section | Component | Example 22 |
| A | Neopentyl glycol dicaprate [%] | 34.8 |
| | Oligoester of Example 2 [%] | 10 |
| | Dipentaerythrityl tripolyhydroxystearate [%] | 5 |
| | Phytosteryl oleate [%] | 5 |
| | Dimethicone [%] | 5 |
| | Tocopherol [%] | 0.2 |
| | Isododecane [%] | 40 |
| Total [%] | | 100 |

<Evaluation Results of Hair Oil>

The hair oil of Example 22 was very compatible with the hair, exhibited a non-sticky sensation during use, passed readily between the fingers, and yielded a finish that had good manageability and was well-arranged with glossiness.

[Example 23] Solid Powder Foundation

A solid powder foundation was produced using the formulation shown in Table 16. The unit "%" in Table 16 indicates "% by mass".

< Raw Materials of Solid Powder Foundation>

As raw materials of the solid powder foundation shown in Table 16, silicon-treated red iron oxide (product name: SI01-2 RED R-516L, manufactured by Daito Kasei Kogyo Co., Ltd.), silicon-treated yellow iron oxide (product name: S101-2 YELLOW LLXLO, manufactured by Daito Kasei Kogyo Co., Ltd.), silicon-treated black iron oxide (product name: SI01-2 BLACK BL-100, manufactured by Daito Kasei Kogyo Co., Ltd.), sericite (product name: Sericite FSE, manufactured by Japan Sericite Corporation), silicon-treated mica (product name: SI-Mica M302, manufactured by Miyoshi Kasei, Inc.), talc (product name: Talc JA-46R, manufactured by Asada Milling Co., Ltd.), polymethyl methacrylate (product name: Matsumoto Microsphere M-100, manufactured by Matsumoto Yushi-Seiyaku Co., Ltd.), squalane (product name: Phytosqualan, manufactured by SOPHIM SAS), dimethicone (product name: KF-96A-20cs, manufactured by Shin-Etsu Chemical Co., Ltd.), and vaseline (product name: NOMCORT W, manufactured by The Nisshin OilliO Group, Ltd.) were used. The titanium oxide was the same as that used in Example 17.

<Production of Solid Powder Foundation>

The components of section A were mixed and dispersed, a mixture prepared by mixing the components of section B under heating at 50°C was added thereto, the resultant was mixed and pulverized, and then the resultant was compression-molded into a metal dish to obtain a solid powder foundation.

[Table 16]

| Table 16 Formulation of solid powder foundation | | | |
|---|---|---|---|
| Section | Component | | Example 23 |
| A | Titanium oxide [%] | | 5 |
| | Silicon-treated red iron oxide [%] | | 0.5 |
| | Silicon-treated yellow iron oxide [%] | | 1.2 |
| | Silicon-treated black iron oxide [%] | | 0.1 |
| | Sericite [%] | | 50 |
| | Silicon-treated mica [%] | | 20 |
| | Talc [%] | | 4.7 |
| | Polymethyl methacrylate [%] | | 2 |
| | Methyl paraoxybenzoate [%] | | 0.5 |
| B | Oligoester of Example 2 [%] | | 10 |
| | Squalane [%] | | 3 |
| | Vaseline [%] | | 1 |
| | Dimethicone [%] | | 2 |
| Total [%] | | | 100 |

<Evaluation Results of Solid Powder Foundation>

The solid powder foundation of Example 23 had an excellent covering effect and a moist feeling with no drying sensation.

[Example 24] Oil-in-Water Emulsion Moisturizing Cream

An oil-in-water emulsion moisturizing cream was produced using the formulation shown in Table 17. The unit "%" in Table 17 indicates "% by mass".

<Raw Materials of Oil-in-Water Emulsion Moisturizing Cream>

As raw materials of the oil-in-water emulsion moisturizing cream shown in Table 17, polyglyceryl-10 stearate (product name: SALACOS PGMSV, manufactured by The Nisshin OilliO Group, Ltd.), polyglyceryl-10 distearate (product name: SALACOS PGDSV, manufactured by The Nisshin OilliO Group, Ltd.), glyceryl stearate (SE) (product name: LASEMUL 92 AE, manufactured by Industrial Quimica Lasem, S.A.U.), carbomer (product name: Carbopol 980, manufactured by Lubrizol Advanced Materials, Inc.), xanthan gum (product name: NOMCORT ZZ, manufactured by The Nisshin OilliO Group, Ltd.), dimethicone (product name: KF-96A-100cs, manufactured by Shin-Etsu Chemical Co., Ltd.), dipentaerythrityl hexa(hydroxystearate/stearate/rosinate) (product name: COSMOL 168ARV, manufactured by The Nisshin OilliO Group, Ltd.), cetanol (product name: KALCOL 6870, manufactured by Kao Corporation), and beeswax (product name: Deodorized Blue Brand Bleached Bees Wax, manufactured by Miki Chemical Industry & Co., Ltd.) were used. The glycerol and BG were the same as those used in Example 21. The squalane was the same as that used in Example 23.

<Production of Oil-in-Water Emulsion Moisturizing Cream>

The components of sections A and B were each stirred and mixed separately under a heated state at 70°C, and the mixture of section B was then added to the mixture of section A and emulsified and dispersed with a homo mixer. After cooling the resultant to 30°C, the component of section C was added thereto and stirred until a uniform mixture was obtained, thereby obtaining an oil-in-water emulsion moisturizing cream.

# EP 4 484 406 A1

[Table 17]

| Table 17 Formulation of oil-in-water emulsion moisturizing cream | | | |
|---|---|---|---|
| Section | Component | | Example 24 |
| A | Polyglyceryl-10 stearate [%] | | 0.8 |
| | Polyglyceryl-10 distearate [%] | | 0.2 |
| | Glyceryl stearate (SE) [%] | | 0.2 |
| | Glycerol [%] | | 5 |
| | BG [%] | | 10 |
| | Methylparaben [%] | | 0.2 |
| | Carbomer [%] | | 0.2 |
| | Xanthan gum [%] | | 0.1 |
| | Water [%] | | 58.8 |
| B | Oligoester of Example 2 | | 10 |
| | Dimethicone [%] | | 0.5 |
| | Squalane [%] | | 2 |
| | Dipentaerythrityl hexa(hydroxystearate/stearate/rosinate) [%] | | 4 |
| | Cetanol [%] | | 2 |
| | Beeswax [%] | | 1 |
| C | 1% Na hydroxide [%] | | 5 |
| Total [%] | | | 100 |

<Evaluation Results of Oil-in-Water Emulsion Moisturizing Cream>

The oil-in-water emulsion moisturizing cream of Example 24 exhibited good skin compatibility, yielded a long-lived moisturizing sensation, and had favorable storage stability.

[Industrial Applicability]

The oligoester according to the present invention can be used as a material of cosmetics, pharmaceutical products, and stationery products.

**Claims**

1.  An oligoester obtained by subjecting a component (A), a component (B) and a component (C) to an esterification reaction, wherein

    the esterification reaction comprises esterifying 0.65 to 0.8 mol of the component (B) and 1.2 to 1.7 mol of the component (C) relative to one mol of the component (A),
    the oligoester has an acid value of 5 mgKOH/g or less, a saponification value of 420 to 510 mgKOH/g, and a hydroxyl value of 60 mgKOH/g or less,
    the component (A) is glycerol,
    the component (B) is a dicarboxylic acid having 2 to 12 carbon atoms, and
    the component (C) is one or more fatty acids selected from the group consisting of caprylic acid, ethylhexanoic acid, isononanoic acid, isopalmitic acid and isostearic acid.

2.  An oligoester obtained by subjecting a component (A), a component (B), a component (C) and a component (D) to an esterification reaction, wherein

    the esterification reaction comprises esterifying 0.65 to 0.8 mol of the component (B) and 1.2 to 1.7 mol of a combined total of the component (C) and the component (D) relative to one mol of the component (A),
a mass ratio between the component (C) and the component (D), component (C) : component (D), used in the esterification reaction is within a range of 99.9:0.1 to 10:90,
the oligoester has an acid value of 5 mgKOH/g or less, a saponification value of 420 to 510 mgKOH/g, and a hydroxyl value of 60 mgKOH/g or less,
the component (A) is glycerol,
the component (B) is a dicarboxylic acid having 2 to 12 carbon atoms,
the component (C) is one or more fatty acids selected from the group consisting of caprylic acid, ethylhexanoic acid, isononanoic acid, isopalmitic acid and isostearic acid, and
the component (D) is one or more fatty acids selected from the group consisting of linear saturated fatty acids having 10 to 24 carbon atoms.

3. The oligoester according to Claim 2, wherein the component (D) is capric acid.

4. The oligoester according to Claim 1 or 2, wherein the component (C) is caprylic acid.

5. The oligoester according to Claim 1 or 2, wherein the component (B) is succinic acid.

6. An oily composition comprising an oligoester of Claim 1 or 2.

7. The oily composition according to Claim 6, further comprising a wax that is solid at 25°C.

8. The oily composition according to Claim 6, wherein the oily composition is a solid.

9. The oily composition according to Claim 6, wherein the oily composition is a semi-solid.

10. A cosmetic comprising an oligoester of Claim 1 or 2.

11. The cosmetic according to Claim 10, further comprising a wax that is solid at 25°C.

12. The cosmetic according to Claim 10, wherein the cosmetic is a lip cosmetic.

13. A stationery product comprising an oligoester of Claim 1 or 2.

14. The stationery product according to Claim 13, wherein the stationery product is a crayon.

15. A method for producing an oligoester, comprising conducting an esterification reaction of 0.65 to 0.8 mol of succinic acid and 1.2 to 1.7 mol of a fatty acid relative to one mol of glycerol, wherein

the fatty acid comprises caprylic acid and capric acid, and a mass ratio between the caprylic acid and the capric acid, caprylic acid : capric acid, is within a range of 99:1 to 10:90, and
the oligoester has an acid value of 10 mgKOH/g or less, a saponification value of 420 to 510 mgKOH/g, and a hydroxyl value of 60 mgKOH/g or less.

16. An oily solid hardness maintenance agent comprising an oligoester of Claim 1 or 2, and a wax that is solid at 25°C.

17. A method for maintaining hardness of an oily solid, comprising incorporating an oligoester of Claim 1 or 2 into an oily solid comprising a wax that is solid at 25°C.

18. A method for improving gloss of an applied surface of a cosmetic, the method comprising incorporating an oligoester of Claim 1 or 2 into the cosmetic.

19. The method for improving gloss according to Claim 18, wherein the cosmetic is a lip cosmetic.

## INTERNATIONAL SEARCH REPORT

| International application No. |
| --- |
| **PCT/JP2023/006436** |

### A. CLASSIFICATION OF SUBJECT MATTER

***C07C 69/40***(2006.01)i; ***A61K 8/37***(2006.01)i; ***A61Q 1/00***(2006.01)i; ***A61Q 17/04***(2006.01)i; ***A61Q 19/00***(2006.01)i;
***C07C 67/08***(2006.01)i; ***C07C 69/30***(2006.01)i; ***C11C 3/02***(2006.01)i
FI: C07C69/40; A61K8/37; A61Q1/00; A61Q19/00; A61Q17/04; C07C69/30; C07C67/08; C11C3/02

According to International Patent Classification (IPC) or to both national classification and IPC

### B. FIELDS SEARCHED

Minimum documentation searched (classification system followed by classification symbols)

C07C69/40; A61K8/37; A61Q1/00; A61Q17/04; A61Q19/00; C07C67/08; C07C69/30; C11C3/02

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

### C. DOCUMENTS CONSIDERED TO BE RELEVANT

| Category* | Citation of document, with indication, where appropriate, of the relevant passages | Relevant to claim No. |
| --- | --- | --- |
| X | JP 06-093288 A (THE NISSHIN OIL MILLS, LTD.) 05 April 1994 (1994-04-05) claims, paragraphs [0007], [0011]-[0013], examples | 2, 4-12, 16-19 |
| Y | | 7, 11, 12, 17-19 |
| A | | 1, 3, 13-15 |
| X | JP 55-104231 A (DYNAMIT NOBEL AG) 09 August 1980 (1980-08-09) claims, page 2, upper right column, line 16 to lower left column, line 7, page 3, upper right column, line 12 to lower left column, line 2, examples | 1-6, 8-10, 15, 16 |
| Y | | 7, 11, 12, 17-19 |
| A | | 13, 14 |
| A | JP 2005-041873 A (GOLDSCHMIDT AG) 17 February 2005 (2005-02-17) entire text | 1-19 |
| A | WO 2021/246862 A1 (PETROLIAM NASIONAL BERHAD) 09 December 2021 (2021-12-09) entire text | 1-19 |

☑ Further documents are listed in the continuation of Box C.       ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **27 April 2023** | **16 May 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**

| International application No. |
| --- |
| **PCT/JP2023/006436** |

**C.     DOCUMENTS CONSIDERED TO BE RELEVANT**

| Category* | Citation of document, with indication, where appropriate, of the relevant passages | Relevant to claim No. |
| --- | --- | --- |
| A | WO 2015/019200 A1 (SILGAN HOLDINGS INC.) 12 February 2015 (2015-02-12) <br> entire text | 1-19 |
| A | US 2017/0137366 A1 (EVONIK DEGUSSA GMBH) 18 May 2017 (2017-05-18) <br> entire text | 1-19 |
| A | WO 2008/013106 A1 (THE NISSHIN OILLIO GROUP, LTD.) 31 January 2008 (2008-01-31) <br> entire text | 1-19 |
| A | JP 59-027807 A (SHISEIDO KK) 14 February 1984 (1984-02-14) <br> entire text | 1-19 |
| A | JP 56-045404 A (SHISEIDO KK) 25 April 1981 (1981-04-25) <br> entire text | 1-19 |
| A | JP 56-032408 A (SHISEIDO KK) 01 April 1981 (1981-04-01) <br> entire text | 1-19 |
| A | AGACH, Mickael et al. Acyl Poly(Glycerol-Succinic Acid) Oligoesters: Synthesis, Physicochemical and Functional Properties, and Biodegradability. Journal of Surfactants and Detergents. 01 August 2016, vol. 19, issue 5, pp. 933-941 <br> entire text | 1-19 |

Form PCT/ISA/210 (second sheet) (January 2015)

INTERNATIONAL SEARCH REPORT
Information on patent family members

International application No.

**PCT/JP2023/006436**

| Patent document cited in search report | | | Publication date (day/month/year) | Patent family member(s) | | | Publication date (day/month/year) |
|---|---|---|---|---|---|---|---|
| JP | 06-093288 | A | 05 April 1994 | US | 5436006 | A | |
| | | | | claims, column 2, lines 34-56, column 3, line 53 to column 5, line 2, examples | | | |
| JP | 55-104231 | A | 09 August 1980 | US | 4366100 | A | |
| | | | | claims, column 1, lines 22-34, column 2, lines 46-57, examples | | | |
| | | | | EP | 14308 | A2 | |
| | | | | DE | 2904164 | A1 | |
| JP | 2005-041873 | A | 17 February 2005 | US | 2005/0031580 | A1 | |
| | | | | EP | 1500427 | A2 | |
| | | | | DE | 10333443 | A | |
| | | | | CN | 1575848 | A | |
| WO | 2021/246862 | A1 | 09 December 2021 | AU | 2021285699 | A1 | |
| WO | 2015/019200 | A1 | 12 February 2015 | US | 2016/0185898 | A1 | |
| | | | | EP | 3030597 | B1 | |
| US | 2017/0137366 | A1 | 18 May 2017 | EP | 3168251 | A1 | |
| | | | | CN | 107011164 | A | |
| WO | 2008/013106 | A1 | 31 January 2008 | US | 2009/0324519 | A1 | |
| | | | | EP | 2048178 | A1 | |
| | | | | CN | 101495538 | A | |
| | | | | KR | 10-2009-0038007 | A | |
| JP | 59-027807 | A | 14 February 1984 | (Family: none) | | | |
| JP | 56-045404 | A | 25 April 1981 | (Family: none) | | | |
| JP | 56-032408 | A | 01 April 1981 | (Family: none) | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2022027108 A
- JP 2009234991 A
- JP 2015124160 A
- JP 2011140479 A
- JP 2007137847 A
- JP 2006315975 A
- JP 7126604 A
- JP 2018168141 A
- JP 2016222637 A
- JP 2015048323 A